# EUROPÄISCHE PATENTSCHRIFT

(11) **EP 1 389 943 B1**
(45) Veröffentlichungstag und Bekanntmachung des Hinweises auf die Patenterteilung: **30.03.2005**
(21) Anmeldenummer: 02747323.0
(22) Anmeldetag: 24.05.2002
(51) Int. Cl.: A61B 3/12, A61B 3/135

(54) **OPHTHALMOSKOP**
OPHTHALMOSCOPE
OPHTALMOSCOPE

(30) Priorität: 25.05.2001 DE 10125596
(43) Veröffentlichungstag der Anmeldung: 25.02.2004
(73) Patentinhaber: Zorn, Wolfgang, 81825 München (DE); Reis, Werner, 80992 München (DE); Spaltmann, Ronald, 83224 Grassau (DE)
(72) Erfinder: Zorn, Wolfgang, 81825 München (DE); Reis, Werner, 80992 München (DE); Spaltmann, Ronald, 83224 Grassau (DE)
(74) Vertreter: Rösler, Uwe, Dipl.-Phys.
(86) Internationale Anmeldenummer: PCT/EP2002/005762
(87) Internationale Veröffentlichungsnummer: WO 2002/094088

(56) Entgegenhaltungen:
- WO-A-97/15855
- US-A- 5 371 557
- US-A- 5 997 141

## Beschreibung

### Technisches Gebiet

Die Erfindung betrifft ein Ophthalmoskop zur Untersuchung des Augenhintergrundes eines Patienten mit wenigstens einer, wenigstens einen Beleuchtungsstrahl erzeugenden Beleuchtungseinrichtung sowie einer der Beleuchtungseinrichtung zuordenbaren Abbildungsoptik, die den Beleuchtungsstrahl über eine Zwischenbildebene auf den Augenhintergrund des Patienten abbildet.
Das Ophthalmoskop weist ferner wenigstens eine Beobachtungseinrichtung sowie eine der Beobachtungseinrichtung zuordenbaren Abbildungsoptik auf, die einen durch Reflexion des Beleuchtungsstrahls am Augenhintergrund erzeugten Beobachtungsstrahl über die Zwischenbildebene in die Beobachtungseinrichtung abbildet. Schließlich ist im Beleuchtungs- und Beobachtungsstrahl eine Blendenanordnung mit je wenigstens einen im Beleuchtungs- und Beobachtungsstrahl einbringaren Blendenspalt vorgesehen, die relativ zu dem Beleuchtungs- und Beobachtungsstrahl synchron schwingend gelagert ist. Eine derartige Ophthalmoskopanordnung wird aufgrund der schwingenden Blendenanordnung, die innerhalb des Beobachtungs- und Beleuchtungsstrahlengangs eingebracht ist, auch als Scanning-Ophtalmoskop bezeichnet.

### Stand der Technik

Eine gattungsgemäße Vorrichtung ist aus der US-PS 3,547,512 bekannt. Durch die spaltförmigen Blenden im Beobachtungsstrahlengang und im Beleuchtungsstrahlengang wird immer nur ein kleiner Bereich des Augenhintergrundes beleuchtet und gleichzeitig beobachtet. Um dennoch einen größeren Teil des Augenhintergrundes untersuchen zu können, werden die Beleuchtungsblende und die Beobachtungsblende synchron bewegt, wodurch ein Teil des Augenhintergrundes abgetastet wird. Wird der Augenhintergrund mit einem Detektor, der eine gewisse Trägheit aufweist beobachtet und erfolgt die Bewegung der Spaltbilder der Blenden schnell genug über immer wieder den gleichen Teil des Augenhintergrundes, dann verschmelzen die Einzelbilder zu einem Gesamtbild des abgetasteten Teiles des Augenhintergrundes.

Diese Vorrichtung soll insbesondere die Betrachtung des Auges durch eine trübe bzw. opake Augenlinse hindurch verbessern, indem die Erzeugung und Beobachtung von Streulicht und die daraus resultierende Blendung vermindert wird. Dabei wird mittels einer Ophtalmoskopierlinse von der Netzhaut ein reelles Zwischenbild außerhalb des Auges erzeugt, wobei die Beleuchtungseinrichtung und das vergrößernde optische System auf die Ebene dieses Zwischenbildes fokusiert sind.

Allerdings ist mit den bisher beschriebenen Maßnahmen alleine noch kein zufriedenstellendes Ergebnis zu erreichen. In der US-PS 3,547,512 werden daher zusätzliche Blenden bzw. Spiegel eingesetzt, die jeweils die Hälfte des Beobachtungs bzw. Beleuchtungsstrahlenbündels ausblenden, sodaß das Beobachtungsstrahlenbündel und das Beleuchtungsstrahlenbündel mit Ausnahme eines kleinen Schnittbereiches in der Objektebene getrennt voneinander verlaufen. Zwar wird dadurch das erzeugte bzw. beobachtete Streulicht erfolgreich weiter verringert, allerdings werden durch die Einschränkungen der Aperturen des Beobachtungsstrahlenbündels und des Beleuchtungsstrahienbündels die Lichtstärke und die erreichbare Auflösung stark verringert.

Eine weitere gattungsgemäße optische Vorrichtung ist der WO 97/15855 zu entnehmen, mit der eine möglichst kontrast- und blendfreie Untersuchung am Augenhintergrund zur Sichtbarmachung feinster Details möglich sein soll. Auch mit dieser Vorrichtung wird der Augenhintergrund mit einem Beleuchtungsstrahl zumindest teilweise ausgeleuchtet und das am Augenhintergrund reflektierte Licht als Beobachtungsstrahl über ein optisches Linsensystem in eine Zwischenbildebene abgebildet, aus der der Beobachtungsstrahl über eine Okulareinheit zur weiteren Analyse abgebildet wird. Die bekannte Anordnung ist zu Zwecken der Kontrasterhöhung ausgelegt worden, indem lichtstarke interferrierende Strahlungsanteile gezielt abgeschwächt werden, sodass auch lichtschwächere Interferenzerscheinungen detektierbar sind. Allerdings bedarf es dennoch einer Okularoptik, zudem ist es nicht möglich zusätzliche Lichtstrahlen in den Beleuchtungs- und Beobachtungsstrahlengang einzukoppeln ohne dabei das optische Gesamtsystem erheblich zu beeinträchtigen.

### Darstellung der Erfindung

Die Aufgabe besteht nun darin einen kompakteren Aufbau für ein Ophthalmoskop mit Schwingblende zu schaffen, also ein Scanning Ophthalmoskop derart auszubilden, das bei einfachen optischen Aufbau eine möglichst direkte Abbildung des Augenhintergrundes auf eine Beobachtungseinrichtung ermöglicht und darüberhinaus die Möglichkeit bietet, weitere Lichtstrahlen in den optischen Aufbau des Ophthalmoskop einzukopplen ohne dabei das optische System nachhaltig zu beeinträchtigen

Die Lösung der Erfindung ist Gegenstand des Anspruchs 1. Vorteilhafte Weiterbildungen der Erfindung sind Gegenstand der Unteransprüche sowie der Beschreibung unter Bezugnahme auf die Ausführungsbeispiele zu entnehmen.

Erfindungsgemäß ist ein Ophthalmoskop nach dem Oberbegriff des Anspruch 1 derart weitergebildet, dass über wenigstens eine optische Einheit die Zwischenbildebene ins Unendliche abbildbar ist, und dass die ins unendliche abgebildete Zwischenbildebene in eine Abbildungsebene des Beobachtungsstrahls abbildbar ist, in der die Beobachtungseinrichtung vorgesehen ist.

Die der Erfindung zugrundeliegende Idee basiert auf der gezielten Verwendung einer optischen Einheit, die die Zwischenbildebene, in der der zu untersuchende Augenhintergrund mit Hilfe einer optischen Abbildungseinheit, vorzusgweise eine Ophtalmoskopierlinse, außerhalb des Auges abgebildet wird, ins Unendliche abbildet, d.h. der Beobachtungsstrahlengang wird in ein parallel verlaufendes Strahlenbündel überführt, der über eine einzige weitere Optikeinheit unmittelbar in eine Abbildungsebene, in der sich die Beobachtungseinrichtung befindet, abgebildet wird. Zum einen können durch diese direkte Strahlführung des Beobachtungsstrahlengangs auf die Beobachtungseinheit jegliche Lichtverluste, die durch zusätzliche weitere Zwischenabbildungsoptiken verbunden wären, ausgeschlossen werden, ebenso wie damit verbundene Abbildungsfehler. Durch diese direkte Abbildung des Beobachtungsstrahls auf eine Beobachtungseinrichtung, die beispielsweise in Form eines CCD-Sensors innerhalb einer Video-Kamera ausgebildet ist, ist es möglich, den optischen Aufbau des Ophthalmoskops sehr klein zu halten und das Ophthalmoskop selbst als Handgerät oder Zusatzmodul beispielsweise für eine Spaltlampe zu verwenden.

Mit Hilfe einer optimal dimensionierten Abbildungsoptik ist es möglich, das komplette Licht, das zur Untersuchung am Augenhintergrund reflektiert wird, über die den Beobachtungsstrahl zugeordneten Abbildungsoptik vollständig, nahezu ohne Verluste, auf die entsprechende Detektorfläche der Beobachtungseinrichtung abzubilden, wodurch die Lichtbelastung für das Patientenauge erheblich reduziert werden kann. Besonders eignet sich der erfindungsgemäße Ophthalmoskopaufbau in Verbindung mit einer Videokamera als Beobachtungseinrichtung in Form eines Video-Scanning-Ophthalmoskops.

Gleichsam dem parallelen, ins Unendliche abgebildeten Beobachtungsstrahlengang, der, wie vorstehend kurz erläutert, über eine, vorzugsweise mehrlinsige Objektivanordnung auf die lichtempfindliche Fläche der Beobachtungseinrichtung abgebildet wird, weist auch der Beleuchtungsstrahlengang einen derartigen parallelen Strahlenabschnitt auf, vorzugsweise parallel verlaufend zum vorstehenden parallelen Strahlengangabschnitt des Beobachtungsstrahls.

Hierzu ist in der Abbildungsoptik des Beleuchtungsstrahls eine Beleuchtungsoptik, vorzugsweise in Form eines Kondensorsystems vorgesehen, das die Lichtstrahlen der Beleuchtungseinrichtung auf eine homogen ausgeleuchtete Fläche, die in der Abbildungsebene der Beobachtungseinrichtung liegt, abbildet. Über eine mehrlinsige Objektivanordnung wird die homogen ausgeleuchtete Fläche in ein parallel verlaufendes Strahlenbündel überführt, das durch die optische Einheit, die den Beobachtungsstrahl ins Unendliche abbildet, in die Zwischenbildebene fokussiert und vermittels der im Strahlengang nachfolgenden optischen Abbildungseinheit auf den Augenhintergrund abgebildet wird.

Im parallel geführten Strahlenabschnitt sowohl des Beobachtungs- als auch des Beleuchtungsstrahlenganges, die beide in diesem Bereich einen minimalen lateralen Abstand zueinander aufweisen und überdies symmetrisch zur optischen Achse der optischen Einheit, das beide Strahlengänge in die Zwischenbildebene abbildet, angeordnet sind, ist es ohne weitere optische Beeinträchtigung der Abbildungseigenschaften des Ophthalmoskops möglich einen weiteren Strahlengang, wie beispielsweise einen Therapielaserstrahl in den Beleuchtungs- und/oder Beobachtungsstrahlengang einzukoppeln. Dies eröffnet einen besonders interessanten Freiheitsgrad bezüglich einer modularen optischen Erweiterung des Ophthalmoskops, wodurch neue Diagnose- und/oder Therapieverfahren am Auge eines Patienten möglich werden.

Die Einfügung weiterer Strahlengänge oder gar optischer Elemente in den parallelen Strahlengang des Beobachtungs- und/oder Beleuchtungsstrahls ist auch in jenen Fällen unkritisch, in denen die optische Einheit zur Abbildung der Zwischenbildebene ins Unendliche axialwärts zu den parallel verlaufenden Beobachtungs- und Beleuchtungsstrahlengänge verschoben wird. Ein axialwärts gerichtetes Verschieben der optischen Einheit kann bspw. aus Gründen eines Ausgleichs der Fehlsichtigkeit des zu untersuchenden Auges durchgeführt werden, wodurch sich zugleich auch die Lage des Fokuspunktes der optischen Einheit ändert. Somit wird stets unabhängig von der Augenfehlsichtigkeit des zu untersuchenden Auges die Zwischenbildebene ins Unendliche abgebildet. Durch Einblenden entsprechender Strichmarken in den Beobachtungsstrahlengang lassen sich definierte Größenmessungen auch bei unterschiedlichen Augenfehlsichtigkeiten am Fundus durchführen. Dies ist von besonderer Wichtigkeit bei Langzeitdokumentationen mit reproduzierbaren Untersuchungsergebnissen. Hierauf wird unter Bezugnahme auf die Ausführungsbeispielse noch im Einzelnen Bezug genommen.

Unmittelbar vor der Abbildungsebene der ausgeleuchteten Fläche sowie vor der Beobachtungseinrichtung, die beispielsweise in Form eines CCD-Sensors ausgebildet ist, befindet sich eine schwingend gelagerte Blendenspaltanordnung, die jeweils einen Blendenspalt für den Beobachtungsstrahl sowie einen Blendenspalt für den Beleuchtungsstrahl vorsieht und die synchron oszillierend die entsprechenden Strahlengänge unterbrechen bzw. freigeben. Die schwingend gelagerte Blendenspaltanordnung dient für eine reflexfreie und kontraststarke Ausleuchtung und Abbildung des Augenhintergrundes. Um unterschiedliche Untersuchungsverfahren am Augenhintergrund, dem sogenannten Fundus, durchführen zu können, ist es notwendig, unterschiedliche breite Blendenspalte einzusetzen. Hierzu weist die Blendenanordnung wenigstens zwei Blendenspalt-Paare auf, die jeweils über unterschiedlich dimensionierte Blendenspaltbreiten verfügen. Durch entsprechende Dimensionierung der Blendenspaltbreiten sowie deren Schwingungsverhalten gegenüber dem Beleuchtungs- sowie Beobachtungsstrahlengang ist es möglich, die Beleuchtungssituation an die jeweils gestellte Messaufgabe am Augenhintergrund individuell anzupassen und zu optimieren.

So bedarf es für angiographische Untersuchungen am Augenhintergrund einer größeren Lichtmenge als im Falle konventioneller Fundusabbildungen. Zur Durchführung bspw. einer Fluoreszenzangiographie sind breite Blendenspalte vorgesehen, die mit einer kleinen Schwingungsamplitude durch den Beobachtungs- sowie Beleuchtungsstrahlengang oszillierend bewegt werden. Werden hingegen Untersuchungen am Augenfundus durchgeführt, so bedarf es einer weit geringeren Beleuchtungsstärke, die durch die Verwendung eines Blendenspalt-Paares mit geringerer Schlitzbreite erhalten wird. Auf weitere Einzelheiten hierzu wird auf eines der nachstehenden Ausführungsbeispiele verwiesen.

Eine weitere Behandlungsmöglichkeit des Augenhintergrundes betrifft die altersbedingte Makuladegeneration, die mit Hilfe der erfindungsgemäßen Vorrichtung höchst präzise untersucht und therapiert werden kann. Für das Auffinden fehlerhafter Stellen in der Netzhaut des Auges wird eine Fluoreszenzangiografie, insbesondere eine Indozyaningrün-Angiografie durchgeführt, bei der der sich in der Netzhaut ablagernde Farbstoff mit Hilfe eines geeigneten Infrarot-Lasers optisch angeregt wird. Dieser IR-Laserstrahl ist über eine geeignete Lichtleiteroptik sowie einen Strahlteiler bzw. Schwenkspiegel in den Beleuchtungsstrahlengang des Ophthalmoskops einbringbar, ohne den übrigen optischen Aufbau des Ophthalmoskops zu beeinträchtigen. Vorzugsweise befindet sich ein derartiger Schwenkspiegel zwischen der Kollimatoroptik und der Beleuchtungseinrichtung, die typischerweise als regelbare Halogenlampe ausgebildet ist. Je nach Anwendungsfall kann auf diese Weise wahlweise Halogenlicht oder IR-Licht längs des Beleuchtungsstrahlenganges eingekoppelt werden. Ebenso ist in dem Beleuchtungsstrahlengang, vorzugsweise längs des Strahlenabschnittes, innerhalb dem, da die Zwischenbildebene ins Unendliche abgebildet ist, ein optisches Sperrfilter einschwenkbar, um je nach Bestrahlungsmodus den von der Halogenlampe beleuchteten Augenhintergrund oder das vom Augenhintergrund ausgehende Fluoreszenzlicht auf die Beobachtungseinheit abzubilden. Nach erfolgter räumlicher Detektion der zu behandelnden Stellen auf der Netzhaut ist es überdies möglich, den Strahl eines Therapielasers zur gezielten Koagulation kleinster Fleckgrößen auf den Augenhintergrund, vorzugsweise in die Feeder-Vessel abzubilden. Hierzu dienen verschiedene Masken, die in die Zwischenbildebene einbringbar sind und gemeinsam mit der optischen Einheit verschiebbar gelagert sind, um bei bestehender Augenfehlsichtigkeit das Abbild der in der Zwischenbildebene eingebrachten Maske scharf auf den Fundus des zu behandelnden Auges abgebildet zu werden. Ebenso können geeignete Durchlichtprojektoren, Filter oder Strichmasken innerhalb der Zwischenbildebene eingebracht werden, um vorab bestimmte, definierte Fundusareale exakt zu markieren.

Um das erfindungsgemäße Ophthalmoskop in seiner Baugröße möglichst klein und kompakt zu halten, ist die Möglichkeit der Einkopplung externer Laserstrahlen im Bereich des parallelen Strahlenganges am zweckmäßigsten. Mittels geeigneter Schnittstellen können externe Laseradapter abnehmbar oder bei Anwendung als Handgerät als Griff verwendet werden. Derartige Laseradapter sind über geeignete Flanschvorrichtungen vorzugsweise schnell und einfach auszutauschen.

Auch gestattet das erfindungsgemäß ausgebildete Ophthalmoskop die stereoskopische Beobachtung des Augenhintergrundes durch Vorsehen zweier räumlich getrennter Beobachtungsstrahlengänge, die entweder mit zwei getrennten Beobachtungseinrichtungen kombiniert sind, oder über eine entsprechende optische Vorkehrung innerhalb der jeweiligen Beobachtungsstrahlengänge zeitlich voneinander getrennt, in periodischer Abfolge als Einzelbildfolge auf eine einzige Beobachtungseinrichtung abbildbar sind.

Weitere, vorteilhafte Eigenschaften sind den folgenden Beschreibungsteilen unter Bezugnahme auf die Ausführungsbeispiele zu entnehmen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Schematische Darstellung der Strahlengänge eines erfindungsgemäßen indirekten Video-Ophthalmoskops mit austauschbarem CCD-Sensor,
- Fig. 2: Optisches System eines Teilstrahlengangs mit austauschbarer optischer Baugruppe in der Zwischenbildeben der Ophthalmoskopierlinse,
- Fig. 3a: Schematische Darstellung einer Therapielaser-Einspiegelung ,
- Fig. 3b: Schematische Querschnittsdarstellung der Therapielaser-Einspiegelung mit Beleuchtungs- und Beobachtungsstrahlengang,
- Fig. 4: Darstellung einer Lasereinspiegelung vor der Ophthalmoskopierlinse,
- Fig 5: Darstellung einer Lasereinspiegelung hinter der Ophthalmoskopierlinse,
- Fig. 6: Schwingend gelagerte Blendenspaltanordnung,
- Fig. 7a, b: Darstellung der schwingenden Blendenspalte,
- Fig. 8a-c: Darstellungen eines optischen Aufbaus eines Stereoophthalmoskops,
- Fig. 9: Darstellung zur Durchführung einer Papillenvermessung,
- Fig. 10a-c: Darstellung zur Durchführung einer ICG-Angiographie am Augenhintergrund und
- Fig. 11 a,b: Einbringen eines Schutzfilters in den Beleuchtungsstrahlengang.

### Wege zur Ausführung der Erfindung, gewerbliche Verwendbarkeit

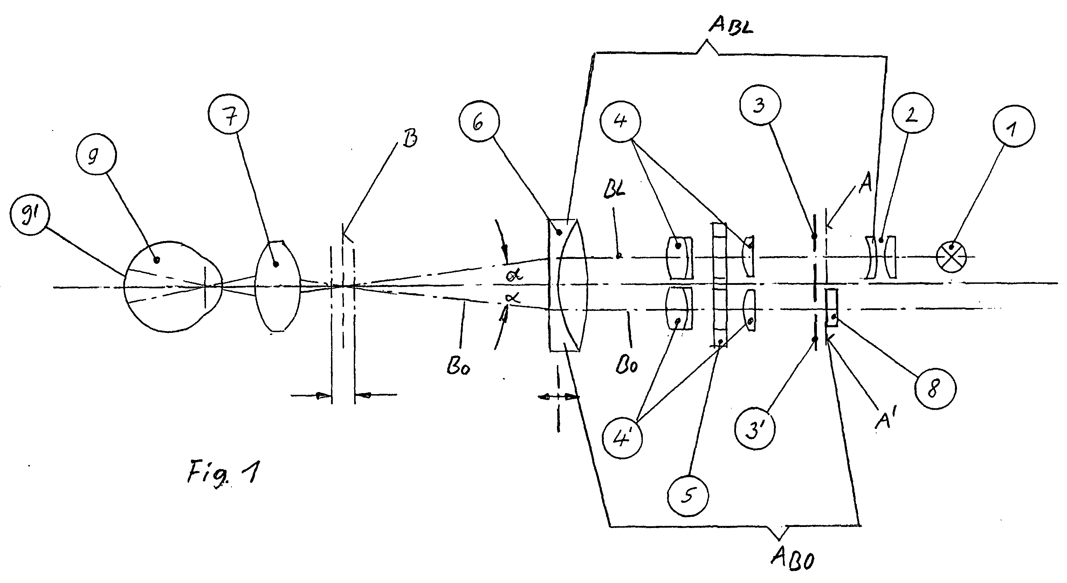

In Fig. 1 ist der optische Grundaufbau eines erfindungsgemäß ausgebildeten Ophthalmoskops dargestellt.

Ausgehend von einer Beleuchtungseinrichtung 1, die als Lichtquelle eine Halogenlampe vorsieht, schließt sich eine aus mehreren optischen Komponenten zusammengesetzte Abbildungsoptik A_{Bl} an, die dem Beleuchtungsstrahl Bl zugeordnet ist. Die Abbildungsoptik A_{Bl} besteht im einzelnen aus einer Kondensoroptik 2, die das Licht der Halogenlampe 1 in eine homogen ausgeleuchtete Fläche in die Abbildungsebene A abbildet, in der eine Blendenspaltanordnung 3 vorgesehen ist, die gegenüber dem Beleuchtungsstrahl BI schwingend gelagert ist und auf die im einzelnen noch eingegangen wird. Im Anschluss an die Blendenspaltanordnung 3 ist in Strahlrichtung eine Optikeinheit 4, in Form eines mehrlinsigen Objektivs vorgesehen, das den Beleuchtungsstrahl BI in einen Parallelstrahl überführt. Über eine optische Einheit 6, die wenigstens ein Akromat aufweist, wird das parallele Beleuchtungsstrahlenbündel in eine Zwischenbildebene B fokussiert, aus der der Beleuchtungsstrahl über eine optische Abbildungseinheit 7, die vorzugsweise als Ophthalmoskopier-Linse ausgebildet ist, auf den Augenhintergrund 9' eines Patienten abgebildet wird.

Am Augenhintergrund 9' wird das vom Beleuchtungsstrahlen BI herrührende Licht reflektiert und in zum Beleuchtungsstrahlengang umgekehrter Strahlrichtung über die optische Abbildungseinheit 7 wieder in die Zwischenbildebene fokussiert, von der das Licht über die, aus mehreren optischen Komponenten bestehende Abbildungsoptik A_{Bo}, die den Beobachtungsstrahlengang Bo des Ophthalmoskops zugeordnet ist, in der nachfolgenden Weise auf eine Beobachtungseinrichtung 8 in Form eines CCD-Sensor abgebildet wird.

Ausgehend von der Zwischenbildebene B, in die der von dem Augenhintergrund 9' herrührende Beobachtungsstrahl Bo fokussiert wird, gelangt der Beobachtungsstrahl Bo auf die optische Einheit 6, die zugleich auch dem Beleuchtungsstrahl Bl als Abbildungsmedium dient und die den Beobachtungsstrahl Bo in ein paralleles Strahlenbündel überführt. Somit dient die optische Einheit 6 der Abbildung der Zwischenbildebene B ins Unendliche. Im Beobachtungstrahlengang folgend ist eine Optikeinheit 4' vorgesehen, die als mehrlinsiges Objektiv ausgebildet ist und der unmittelbaren Abbildung des Beobachtungsstrahls Bo in die Abbildungsebene A' dient, in der sich die lichtempfindliche Detektorfläche der Beobachtungseinrichtung 8 befindet. Im Beobachtungsstrahlengang Bo zwischen der Optikeinheit 4' und der Beobachtungseinrichtung 8 ist ebenso ein Spalt der Blendenspaltanordnung 3' vorgesehen, der gleichsam dem Blendenspalt im Beleuchtungsstrahlengang schwingend gelagert ist. Die Blendenanordnung 3, 3' ist vorzugsweise, wie im weiteren noch im einzelnen dargelegt wird, als Blendenspaltpaar ausgeführt, das zeitsynchron innerhalb des Beleuchtungs- und Beobachtungsstrahlengangs schwingt.

In Kombination mit den Optikeinheiten 4, 4' ist eine Filteranordnung 5 vorgesehen, die in die jeweiligen Strahlengänge des Beobachtungs- sowie Beleuchtungsstrahls herausnehmbar einbringbar ist. Der laterale Abstand zwischen dem Beleuchtungs- und Beobachtungsstrahlengang ist insbesondere im Bereich des Strahlabschnittes, in dem die Zwischenbildebene B ins Unendliche abgebildet ist und somit die jeweiligen Strahlengänge parallele Strahlenbündel aufweisen, sehr klein gewählt, um einerseits den optischen Aufbau möglichst klein und kompakt auszugestalten und andererseits die Abbildung beider Strahlengänge mit Hilfe nur einer einzigen, als Vorsatzlinse ausgebildeten optischen Einheit 6 zu realisieren. Auf diese Weise ist es möglich, dass durch axiales Verschieben der optischen Einheit 6 in Bezug auf die gemeinsame optische Achse A des Beleuchtungs- und Beobachtungsstrahlenganges eine etwaige Fehlsichtigkeit des Auges des zu behandelnden Patienten ausgeglichen werden kann. Typischerweise ist es möglich, den Verschiebebereich der optischen Einheit 6 beidseitig zu einer Normlage derart so zu wählen, dass Fehlsichtigkeiten von ± 15 dpt kompensiert werden können. Mit Hilfe einer nicht in der Figur 1 dargestellten Dioptrieskala, die beispielsweise innerhalb der Zwischenbildebene B einbringbar ist, ist eine definierte Voreinstellung der Augenfehlsichtigkeit vornehmbar.

Da die als Ophthalmoskopier-Linse ausgebildete optische Abbildungseinheit 7 gleichsam der optischen Einheit 6 sowohl vom Beleuchtungs- als auch vom Beobachtungsstrahlengang durchsetzt wird, wird aufgrund der symmetrischen Strahlführung die Beobachtungs-Abbildungsebene A', in der sich die lichtempfindliche Detektorfläche, beispielsweise ein CCD-Sensor, der Beobachtungseinrichtung 8 befindet, als auch die Beleuchtungs-Abbildungsebene A, die als homogen ausgeleuchtete Fläche erscheint, gemeinsam auf den Augenhintergrund 9' des zu untersuchenden Auges 9 abgebildet und zur Deckung gebracht werden.

Zur blend- und reflexionsfreien Beobachtung des Augenhintergrundes mit Hilfe des in Fig. 1 dargestellten Ophthalmoskops werden die Blendenspalte, die jeweils in den Beleuchtungs- und Beobachtungsstrahlengang eingebracht sind, synchron zueinander relativ zu dem Beleuchtungs- und Beobachtungsstrahl in oszillierender Weise bewegt. Hierdurch erfährt der Beleuchtungs- und Beobachtungsstrahlengang zeitsynchrone Unterbrechungen, so dass einzelne, kurzfristige Momentaufnahmen vom beleuchteten Augenhintergrund möglich wird. Jede einzelne Momentaufnahme erfasst jedoch nur einen Teilbereich des Augenhintergrundes, dessen Lage und Größe durch die jeweilige Blendenspaltbreite, die jeweils für beide Blendenspalte im Beobachtungs- und Beleuchtungsstrahlengang identisch gewählt sind, sowie die jeweilige aktuelle Position der Spalte in Bezug auf den Beleuchtungs- und Beobachtungsstrahlengang abhängt.

Grundsätzlich lassen sich alle in Fig. 1 dargestellten Komponenten des Ophthalmoskops je nach gewünschten Aufnahmebedingungen des Augenhintergrundes austauschen und aufeinander abstimmen. Insbesondere ist es möglich, die als Ophthalmoskopier-Linse ausgebildete optische Abbildungseinheit 7 zusammen mit der optischen Einheit 6 auszutauschen, um einen gewünschten Bildfeldwinkel am Augenhintergrund zu erhalten. Auch ist die Beobachtungseinrichtung 8 über eine entsprechende leicht lösliche Flanschstruktur gegen alternative Beobachtungseinrichtungen austauschbar. So können beispielsweise Farbkameras leicht gegen hochempfindliche Schwarzweiß-Kameras, beispielsweise für die Durchführung von Fluoreszenzangiografieaufnahmen ausgetauscht werden. In diesem Zusammenhang ist es ebenso vorteilhaft, die Blendenanordnung 3, 3' in Abhängigkeit der verwendeten Kamera, insbesondere der Flächengröße der lichtempfindlichen Detektoreinheit, auszuwählen und anzupassen. So bedarf es beispielsweise bei der Durchführung der Fluoreszenzangiografie größerer Spaltbreiten, als im Falle konventioneller Fundusuntersuchungen am Augenhintergrund.

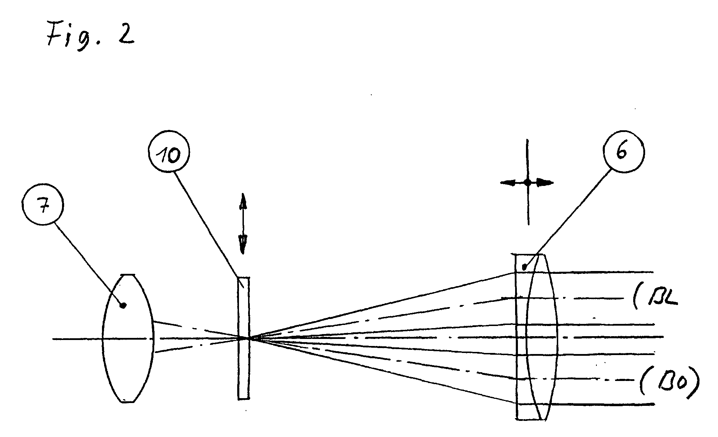

In Fig. 2 ist ein Teilstrahlengang des Ophthalmoskops dargestellt, bestehend aus der als Opthalmoskopier-Linse ausgebildeten optischen Abbildungseinheit 7, einer innerhalb der Zwischenbildebene B beliebig einsetzbar bzw. austauschbaren optischen Baugruppe 10 sowie der axial verschieblichen optischen Einheit 6, durch die sowohl der Beleuchtungs- (Bl) als auch Beobachtungsstrahlengang (Bo) hindurchtreten. Die austauschbare Baugruppe 10 besteht beispielsweise aus einer feststehenden Glasplatte ohne Markierungen, einer austauschbaren Glasplatte mit aufgebrachten Markierungen oder Skalen, einer Filterbaugruppe, einer austauschbaren Maske, beispielsweise für bestimmte abgegrenzte Bestrahlungsgebiete an der Retina, einen Durchlicht-Diaprojektor, der ebenfalls zur Vorauswahl bestimmter zu bestrahlender Flächen am Fundus dient oder einer Dioptrie-Skala. Vorzugsweise ist die optische Baugruppe 10 mit der optischen Einheit 6 mechanisch derart gekoppelt, so dass sie gemeinsam längs in Axialrichtung zur Einstellung der Fehlsichtigkeit des zu untersuchenden Auges verschiebbar gelagert ist.

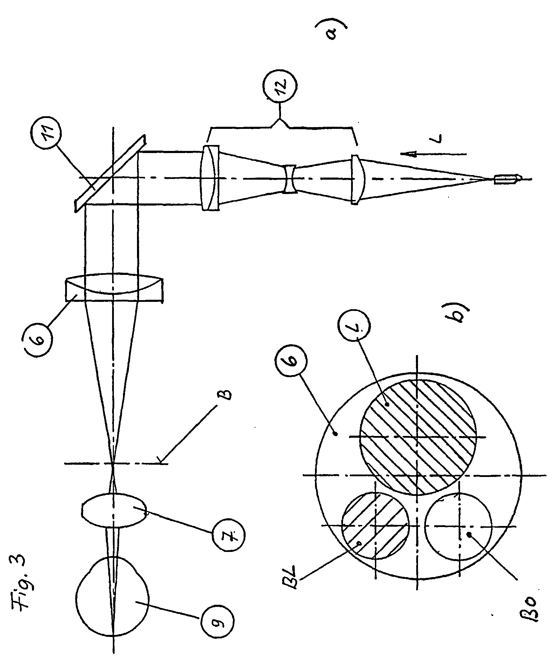

In Fig. 3a ist eine schematisierte Darstellung eines Teilstrahlenganges des erfindungsgemäß ausgebildeten Ophthalmoskops mit Therapielasereinspiegelung gezeigt. Um das Anwendungsgebiet des indirekten Video-Ophthalmoskops zu erweitern, wird über eine zusätzliche Umlenkoptik, beispielsweise in Form eines Spiegels 11, eine Einkopplung eines Therapielasers 12 ermöglicht. In vorteilhafter Weise befindet sich das als Spiegel ausgebildete Einkoppelelement 11 in Strahlrichtung des Beleuchtungsstrahlenganges BI vor der optischen Einheit 6, also im Bereich, in dem der Beleuchtungs- sowie auch Beobachtungsstrahlengang parallele Strahlenbündel darstellen. Auf diese Weise gelangt der durch die optische Einheit 6 erzeugte Fokuspunkt des Therapielasers stets in die Zwischenbildebene B unabhängig von der aktuellen axialen Lage der optischen Einheit 6. Die sich auf dem Augenhintergrund abbildende Fleckgröße des Laserspots kann über eine entsprechende Fokussieroptik, die durch die Abbildungseigenschaften der optischen Abbildungseinheit 7 sowie die Abbildungsverhältnisse des Auges gegeben ist, selbst angepasst werden.

Zur räumlichen Lageveränderung des am Augenhintergrund abgebildeten Laserspots ist der Umlenkspiegel 11 vorzugsweise in X- und Y-Richtung verschwenkbar in Form eines Scanning-Spiegels gelagert.

Aus Gründen einer verbesserten Bedienungsfreundlichkeit des Ophthalmoskops lässt sich der Therapielaser 12 als Adaptereinheit derart ausführen, so dass der Therapielaser über eine geeignete, am Gehäuse des Ophthalmoskops vorgesehene Schnellverschluss-Schnittstelle anbringbar ist. An dieser Schnittstelle kann beispielsweise ein Handgriff angebracht sein, der gegen den Therapielaser-Adapter austauschbar ist.

In Fig. 3b ist ein Schnittbild durch die einzelnen Strahlengänge dargestellt, im Bereich der ins Unendliche abgebildeten Zwischenbildebene. In dieser Ansicht wird die Aufteilung der einzelnen Strahlengänge, d.h. des Beleuchtungs-(BI), Beobachtungs- (Bo) sowie Therapielaser-Strahlengang (L) deutlich. Die einzelnen Strahlengänge werden gemeinsam über die optische Einheit 6 über die Zwischenbildebene B und der optischen Abbildungseinheit 7 auf den Augenhintergrund abgebildet.

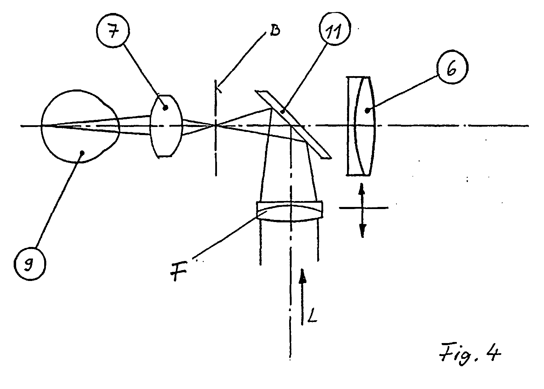

In Fig. 4 ist ein Ausführungsbeispiel eines Teilbereiches des Ophthalmoskops dargestellt, bei dem die Therapielaser-Einkopplung zwischen der Zwischenbildebene B und der optischen Einheit 6 erfolgt. Bei entsprechender Lageveränderung der optischen Einheit 6 aus Gründen der Anpassung an die Fehlsichtigkeit des zu behandelnden Auges 6 ist dafür zu sorgen, dass auch der Fokuspunkt des Therapielaserstrahls geeignet korrigiert wird. Dies erfolgt in dem in Fig. 4 dargestellten Ausführungsbeispiel mit Hilfe einer Fokussierlinse F.

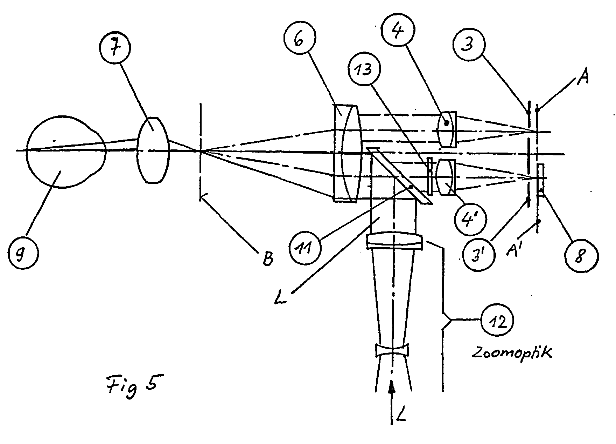

In Fig. 5 ist schließlich die Situation der optischen Einkopplung eines Therapielaserstrahl L dargestellt, in der mit Hilfe eines als Umlenkspiegels ausgebildeten Umlenkelementes 11 der Therapielaserstrahl L ausschließlich längs zum Beobachtungsstrahlengang Bo im Bereich, in dem die Zwischenbildebene B ins Unendliche abgebildet ist, eingekoppelt wird. Um zu verhindern, dass der Therapielaserstrahl L auf die lichtempfindliche Fläche der Beobachtungseinheit 8 gelangt, ist entweder der Umlenkspiegel 11 wellenlängenselektiv ausgebildet, d.h. nicht durchlässig für die Therapielaser-Wellenlänge, oder ein zusätzliches Schutzfilter 13 ist entsprechend im Strahlengang des Beobachtungsstrahls Bo eingebracht.

In gleicher Weise ist es ebenso möglich, bei festem Verbleib des Schutzfilters 13 die Therapielaserstrahl-Einkopplung über ein, im Beleuchtungsstrahlengang Bl eingebrachtes Umlenkelement 11 vorzunehmen.

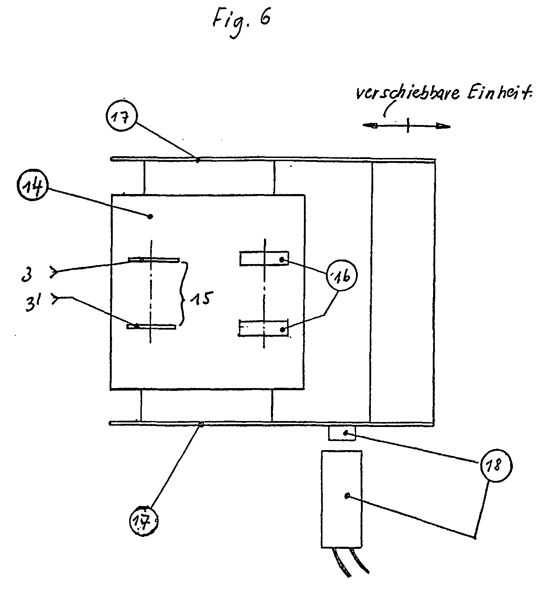

In Fig. 6 ist eine schematisierte Darstellung der schwingend gelagerten Blendenspaltanordnung 3, 3' dargestellt. Die Blendenspaltanordnung ist im wesentlichen aus einem lichtundurchlässigen Material, beispielsweise einem Flachblech-Material 14 gefertigt, das zwei Blendenspalt-Paare 15, 16 aufweist, die jeweils untereinander identische Blendenspaltbreiten vorsehen. Das Blendenspalt-Paar 15 weist gegenüber dem Blendenspalt-Paar 16 kleinere Blendenspaltbreiten auf. Die Blendenspaltanordnung 14 ist gegenüber dem Beleuchtungs- und Beobachtungsstrahlengang derart angeordnet, dass im gezeigten Ausführungsbeispiel gemäß Fig. 6 der untere Blendenspalt 3' des Blendenspalt-Paares 15 gegenüber dem Beobachtungsstrahlengang Bo schwingt, wohingegen zeitsynchron der obere Blendenspalt 3 des Blendenspalt-Paares 15 gegenüber dem Beleuchtungsstrahlengang BI schwingt. Der Schwingvorgang der Blendenspaltanordnung 14, der im gezeigten Ausführungsbeispiel vertikal von oben nach unten und umgekehrt erfolgt, wird durch eine geeignete Federlagerung 17, die über einen geeigneten Induktionsmechanismus 18 in oszillierender Weise ausgelenkt wird, realisiert. Zusätzlich ist die Blendenspaltanordnung 14 horizontal verschieblich gelagert, so dass durch entsprechend seitliches Verschieben der Blendenspaltanordnung das Blendenpaar 16 in den jeweiligen Strahlengang des Beleuchtungs- und Beobachtungsstrahlengang positionierbar ist.

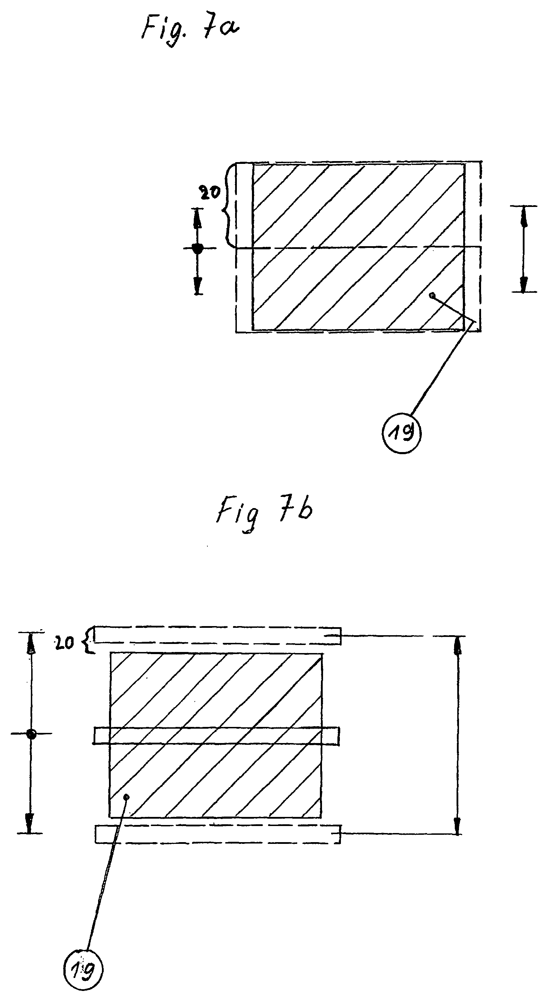

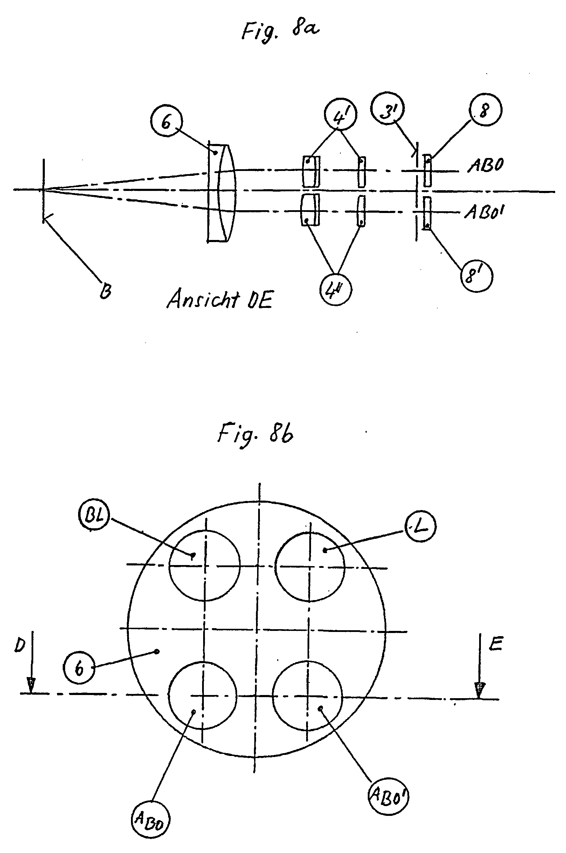

Von besonderer Bedeutung ist die in Bezug auf Fig. 6 angesprochene vertikale Schwingbewegung für die jeweils angewandte Untersuchungstechnik, mit der der Augenhintergrund analysiert wird. Gilt es beispielsweise an dem zu untersuchenden Augenhintergrund eine Fluoreszenzangiografie, beispielsweise eine Indozyaningrün-Angiografie, durchzuführen, so gilt es zur Fluoreszenzanregung auf dem Augenhintergrund eine große Lichtintensität zu deponieren. Bei herkömmlichen Ophthalmoskopen werden hierfür lichtstarke Xenon-Blitzröhren mit Schnellblitzgeneratoren oder leistungsstarke Beleuchtungslaser eingesetzt, die einen großen technischen sowie auch finanziellen Aufwand erfordern. Im Gegensatz hierzu ist es mit dem erfindungsgemäßen Ophthalmoskop möglich, übliche, für Funduskameraaufnahmen ausreichende, in der Helligkeit regelbare Halogenlampen einzusetzen, wobei zur Fluoreszenzanregung eine Blendenanordnung eingesetzt wird, deren Blendenspalte sehr viel größer sind als typische Blendenspaltdimensionen, die für Fundusaufnahmen geeignet sind. So wird in besonders vorteilhafter Weise für die Fluoreszenzangiografie in den Beobachtungs- und Beleuchtungsstrahlengang ein Blendenspalt-Paar eingesetzt, dessen einzelne Blendenspalte eine Blendenspaltbreite aufweisen, die vorzugsweise die Hälfte jener Flächenerstreckung der lichtempfindlichen Fläche der Beobachtungseinrichtung beträgt, längs der der Blendenspalt oszillierend bewegt wird. Im einzelnen geht dies aus Fig. 7a hervor, in der angenommen sei, dass die lichtempfindliche Fläche 19 des CCD-Sensors rechteckförmig ausgebildet ist, längs der sich gemäß Pfeildarstellung der Blendenspalt 20 oszillierend bewegt. Die oszillierende Bewegung des Blendenspaltes 20 gegenüber der lichtempfindlichen Fläche 19 erfolgt derart, dass die gesamte Blendenspaltbreite stets innerhalb der lichtempfindlichen Fläche 19 verbleibt, d.h. der Umkehrpunkt für die oszillierende Bewegung des Blendenspaltes ist genau so gewählt, dass die Blendenspaltbreite sich stets innerhalb der lichtempfindlichen Fläche 19 befindet. Auf diese Weise wird sichergestellt, dass das gesamte Licht der Beleuchtungseinrichtung, das vom Augenhintergrund reflektiert wird, auf die lichtempfindliche Fläche 19 der Beobachtungseinrichtung auftrifft. Somit ist für eine optimale Lichtausnutzung gesorgt, wodurch der Einsatz kostspieliger, lichtstarker Lichtquellen vermeidbar wird.

Im umgekehrten Falle der Fundusbeobachtung wird das in Fig. 6 dargestellte Blendenspalt-Paar 15 in den Beobachtungs- und Beleuchtungsstrahlengang geschoben, wobei gemäß Fig. 7b der sehr schmale Blendenspalt 20 derart gegenüber der lichtempfindlichen Fläche 19 oszillierend bewegt wird, dass der Umkehrpunkt des Blendenspaltes 20 jeweils außerhalb der lichtempfindlichen Fläche 19 liegt.

Mit Hilfe der vorstehend geschilderten Blendenanordnung ist es möglich, mit Hilfe eines einzigen Scanning-Video-Ophthalmoskops Fundusuntersuchungen am Augenhintergrund sowie auch Fluoreszenzangiografie-Aufnahmen durchzuführen. Hierdurch eröffnen sich vollkommen neue Möglichkeiten und Wege für die Untersuchung des Auges am Augenhintergrund.

Ebenso bietet das erfindungsgemäß ausgebildete Scanning-Ophthalmoskop die Möglichkeit einer stereoskopischen Betrachtung des Augenhintergrundes für eine räumlich aufgelöste Diagnose. So ist es für die Fundusdiagnose oftmals hilfreich, stereoskopische, d.h. dreidimensionale Bilder vom Augenhintergrund zu erhalten.

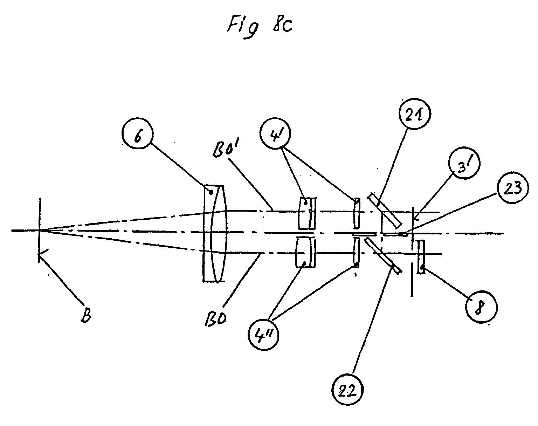

Hierzu sieht das Ausführungsbeispiel gemäß Fig. 8a, das lediglich eine Teildarstellung der Strahlengänge des Ophthalmoskopes zeigt, zwei parallel geführte Beobachtungsstrahlengänge mit den zugehörigen Abbildungsoptiken A_{BO} sowie A_{BO'} mit jeweils getrennten Beobachtungseinrichtungen 8 bzw. 8'. Demgegenüber ist in Fig. 8b eine Querschnittsdarstellung durch die optische Einheit 6 dargestellt, in der alle Strahlengänge des Ophthalmoskopes zur stereografischen Bildaufnahme dargestellt sind. So sei angenommen, dass in der oberen Hälfte der optischen Einheit 6 die Strahllage des Beleuchtungsstrahls Bl sowie beispielsweise eines zusätzlich eingekoppelten Therapielaserstrahls L vorgesehen sind, wohingegen in der unteren Hälfte die beiden, getrennten Beobachtungsstrahlengänge A_{Bo} sowie A_{Bo'} gemäß Fig. 8a vorgesehen sind.

In gleicher Weise ist es möglich, einen stereoskopischen Seheindruck mit nur einer einzigen Beobachtungseinrichtung zu erhalten, indem gemäß Fig. 8c die beiden getrennt voneinander vorgesehenen Beobachtungsstrahlengänge Bo sowie Bo' über einen Umlenkspiegel 21 bzw. Teilerspiegel 22 auf die Beobachtungseinheit 8 umgelenkt werden. Eine Shutter-Blende 23 sorgt für einen repitierenden Bildwechsel zwischen beiden Beobachtungsstrahlengängen auf der Beobachtungseinrichtung 8, in der die nacheinander von beiden getrennten Beobachtungsstrahlengängen Bo, Bo' herrührenden Einzelbilder in geeigneter Weise in Überlagerung zur dreidimensionalen Bilddarstellung gebracht werden. Durch diese stereoskopische Betrachtungsweise kann die Therapie sowie die Diagnosedarstellung am Fundus, aber insbesondere auch im vorderen Augenmedium erheblich gegenüber bisher bekannten Techniken verbessert werden.

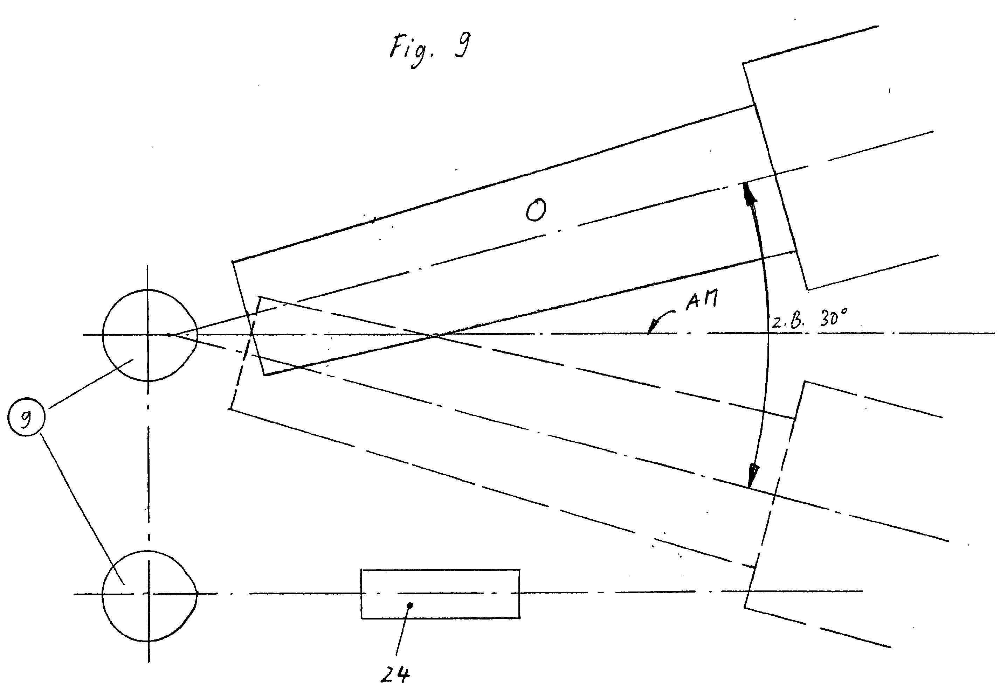

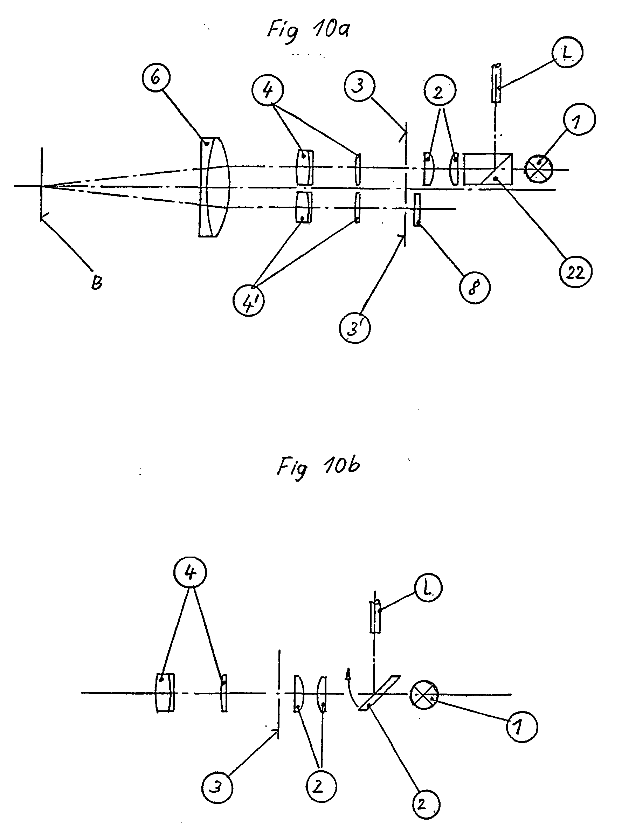

Zur Untersuchung des Glaukoms (grüner Star) am Auge ist eine genaue Analyse, speziell die Vermessung des Sehnervenknotens, die sogenannte Papille, erforderlich. Hierbei wird die Papille mit normalen Referenzbildern verglichen, wodurch Veränderungen an der Papille dokumentiert und ausgewertet werden. Um möglichst eine genaue topographische Darstellung der Papille zu erhalten, ist eine stereoskopische Einzelbildauswertung erforderlich. Hierbei wird zur Stereoaufnahme das Ophthalmoskop in einer horizontalen Ebene gegenüber dem zu untersuchenden Auge um einen definierten Winkel, vorzugsweise 30°, verschwenkt. Damit der Patient während beider Aufnahmesituationen eine fest vorgegebene Blickrichtung einhält, ist das Ophthalmoskop mit einer optischen Markiereinheit 24 ausgerüstet, die unabhängig von der verschwenkten Position des Ophthalmoskops eine feste, unveränderliche Blickrichtung für den Patienten definiert. Eine diesbezügliche Untersuchungssituation ist in Fig. 9 schematisch dargestellt.

Es sei angenommen, dass die Zeichenebene gemäß Fig. 9 eine Horizontalebene darstellt, in der das zu untersuchende Auge 9 in der Draufsichtdarstellung gezeigt ist. Das Ophthalmoskop O befindet sich in einem definierten Abstand vom Auge 9 und ist bezüglich einer Mittenachse AM jeweils um einen fest definierten Winkelbetrag, vorzugsweise β = 30° verschwenkbar. In den jeweils angegebenen Aufnahmepositionen des Ophthalmoskops O werden Aufnahmen vom Augenhintergrund, insbesondere zur Papillenaufnahme angefertigt, die im weiteren zur stereografischen Bildauswertung zusammengefügt werden. Um zu gewährleisten, dass das Patientenauge 9 während beider Aufnahmesituationen eine unveränderte Blickrichtung längs der Mittenachse AM beibehält, ist eine Markiereinrichtung 24 ortsfest am Ophthalmoskop oder im Bereich des Ophthalmoskops vorgesehen, die je nach Aufnahmeposition die fest vorgegebene Blickrichtung für den Patienten anzeigt.

In Kombination zur vorstehend beschriebenen, stereoskopischen Bildauswertung der Papille für die Glaukomuntersuchung ist es zusätzlich erforderlich, die Papille im Wege einer messtechnischen Tiefenerfassung zu analysieren, wodurch das Volumenmaß der Papillenaushöhlung exakt bestimmt werden kann, um letztlich ein Indiz für den Fortschritt des grünen bzw. grauen Stars zu erhalten.

Hierfür wird vorgeschlagen, das gesamte Ophthalmoskop oder vorzugsweise ausschließlich die optische Einheit 6 des Ophthalmoskops längs zur Blickrichtung ortsaufgelöst zu verschieben, um auf diese Weise Einzelbilder aus verschiedenen Tiefen in Art einzelner Schnittbildaufnahmen zu erhalten. Die Ansteuerung der Lageveränderung der optischen Einheit 6 bzw. des gesamten Ophthalmoskops erfolgt vorzugsweise mit Hilfe eines Schrittmotors, der die Lageverschiebung in definiert vorgegebenen Einzelschritten vornimmt. Die aus einzelnen Tiefenebenen aufgenommenen Einzelbilder werden mit der Video-Einzelbildaufnahme synchronisiert und mittels einer geeigneten Bildauswerte-Software zu einem einzelnen dreidimensionalen topografischen Abbild der Papille zusammengefügt.

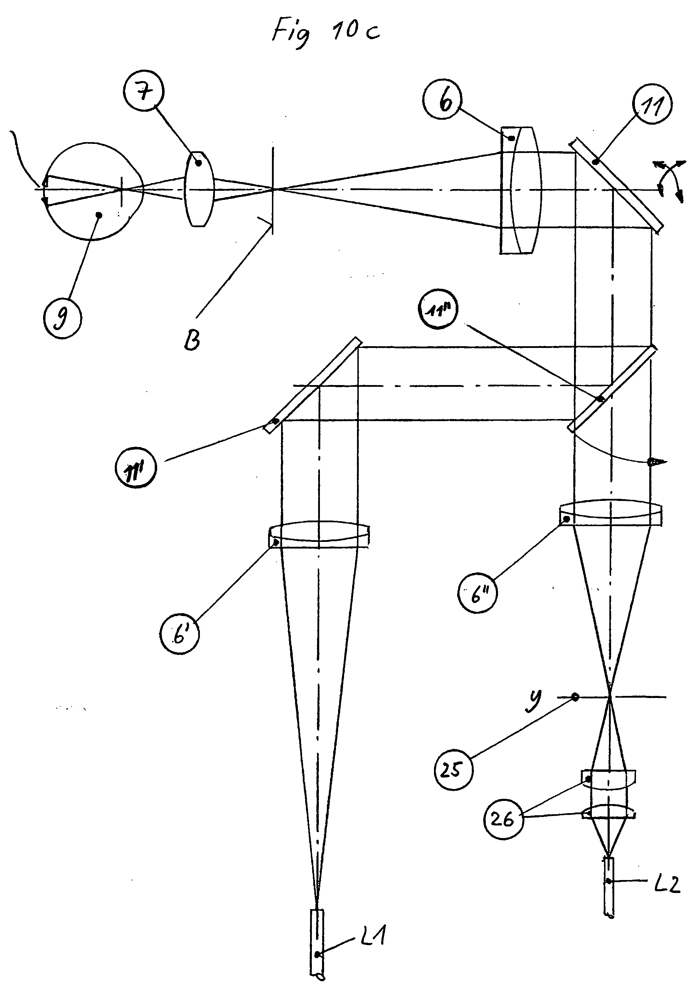

Das erfindungsgemäße Ophthalmoskop bietet ferner eine vorteilhafte Kombination aus Diagnose- und Therapiemaßnahmen, die für eine erfolgreiche Behandlung der altersbedingten Makuladegeneration (AMD) einsetzbar sind.
Bei diesem Typ entsteht eine Barrierenstörung zwischen der Netzhaut und den darunterliegenden Pigment- und Gefäßschichten. Eine gefäßhaltige , neovaskuläre Narbe wächst in die Netzhautmitte ein und führt zu einer fortschreitenden Zerstörung der Photorezeptor- Sinneszellenschicht, die im schlimmsten Fall zur vollständigen Erblindung führen können.
Zur Diagnose derartiger Neovaskularisationen, wird eine Fluoreszenz (FAG)- oder Indozyaningrün (ICG)-Angiografie durchgeführt, die die erkrankten Fundusareale oder Gefäße mit Hilfe des Farbstoffes sichtbar machen. Zur Farbstoffanregung bedarf es einer starken Lichtquelle, vorzugsweise im blauen (FAG) oder im infraroten (ICG) Spektralbereich, die den Fluoreszenzfarbstoff optisch anzuregen vermögen. Gemäß eines bevorzugten Ausführungsbeispiel des Ophthalmoskops, dessen schematisierter Aufbau in Figur 10 a dargestellt ist und im wesentlichen über die bereits in Figur 1 dargestellten Komponenten verfügt, auf die nicht weiter eingegangen wird, ist zwischen der Beleuchtungseinrichtung 1 und der Kondensoroptik 2 ein Strahlteiler 22 vorgesehen, der Licht eines emittierenden Lasers L, bspw. mit einer Wellenlänge von 480 nm oder 780 nm, in den Beobachtungsstrahlengang einkoppelt. Alternativ ist es möglich gemäß Figur 10 b das Laserlicht L über einen schwenkbaren Umlenkspiegel 21 in den Beobachtungsstrahlengang einzukoppeln. Letztere Variante ermöglicht eine abwechselnde Beleuchtung des Augenhintergrundes mit Laserlicht oder Licht der Lichtquelle 1.

Im Wege der vorstehenden Diagnose werden mit Hilfe von Angiografieaufnahmen genaue Informationen über die räumliche Lage und Größe der Neovaskularisationen gewonnen. Diese Aufnahmen dienen im Weiteren durch Überlagerung mit einer nachfolgenden Beobachtung des Augenhintergrundes, bei der geeignete FAG oder ICG-aufnahmen unter Einsatz der Technik des Eye-Trackings mit dem Abbild des Augenhintergrundes in Deckung gebracht werden, einer genauen Identifikation defekter, zu therapierender Netzhautbereiche, die mit einer erweiterten Ophthalmoskopanordnung gemäß der Figur 10 c behandelbar sind.

In Figur 10 c ist ein Teil der Optik des Ophthalmoskops dargestellt, in dessen Strahlengang, in dem die Zwischenbildebene B ins Unendliche abgebildet ist, ein Schwenkspiegel 11 eingebracht ist. Über den Schwenkspiegel 11 lassen sich zwei getrennte Lasersysteme L1, L2 in den Strahlengang des Ophthalmoskops einblenden, mit denen eine unmittelbare Therapie der diagnostizierten altersbedingten Makuladegeneration möglich ist.

So wird über die Umlenkspiegel 11', 11" und 11 das Licht eines Therapielasers L1 mit einer Wellenlänge von 810 nm in den Strahlengang eingekoppelt, der mit einer Fleckgröße von 50 oder 100 µm Durchmesser auf den Augenhintergrund fokussiert wird. Mit Hilfe eines derartigen Lichtfleckes ist es möglich, kleinste Netzhautbereiche zu koagulieren, so insbesondere Bereiche, in denen die sogenannte "Feeder-Vessel" verläuft, die als Ursache für die Ausbildung der AMD angesehen werden kann. Zur exakten Lokalisierung dieser Bereiche dienen die im Vorfeld aufgenommenen FAG- oder ICG-aufnahmen, die mit dem aktuellen Augenhintergrund anhand charakteristischer Deckungspunkte in konforme Überlagerung gebracht werden. Zur Ausrichtung des Laserstrahls können geeignete Fokuszieleinrichtungen eingesetzt werden oder geeignet designte Masken werden in den Strahlengang des Lasers, bspw. in die Zwischenbildebene B eingebracht.

Zur optischen Einkopplung des Laserstrahls des Lasers L1 in den Strahlengang des Ophthalmoskops dient eine, neben den erwähnten Spieglen 11, 11' 11", den divergent verlaufenden Laserstrahl in einen Parallelstrahl umformende Linseneinheit 6'.

Ein zweiter Laser L2 dient zur Erzeugung von Laserstrahlung mit einer Wellenlänge von 690 nm, der für eine großflächigere Bestrahlung, bspw. mit einer Fleckgröße von 400 µm bis 6000 µm, des Augenhintergrundes 9' geeignet ist. Im Strahlengang des Lasers L2 ist eine Abbildunsgeinheit 26 vorgesehen, die eine Fokalebene y aufweist, in die bspw. optische Masken 25 zur gezielten Fleckgestaltung, einbringbar sind. Auch können Irisblenden oder Displays in diese Ebene y je nach geeigneten Abbildungswünschen eingesetzt werden. Wieder bildet eine Linseneinheit 6" den divergenten Laserstrahl zur weiteren Einkopplung in einen paralleleln Strahl um.

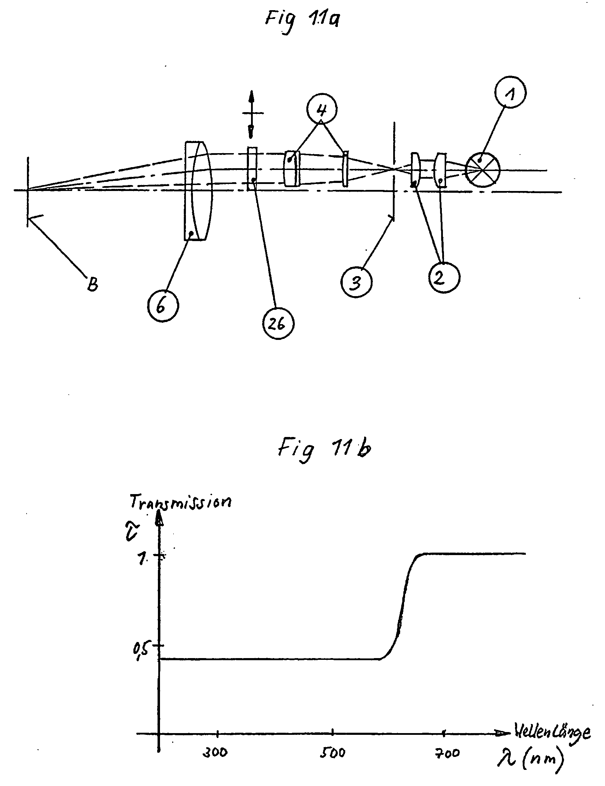

Von großem Vorteil ist bei der Durchführung einer derartigen Behandlung zur Bekämpfung der Diabetes unter Verwendung eines Langpassinterferenzfilters, vorzugsweise eines RG-6-Filters, der über eine konstante niedrige Transmission im sichtbaren Bereich verfügt, jedoch den IR-Anteil des Halogenlampenlichts voll durchlässt. Der Filter ist vorzugsweise im Beleuchtungsstrahlengang vor der optischen Einheit 6 vorzusehen, wodurch die durch die variierende Lichtbelastung entstehende Unruhe am Patientenauge entscheidend gemindert werden kann. Durch Vorsehen eines derartigen Filters ist es möglich, während der Lasertherapie den sichtbaren Lichtanteil zu reduzieren, gleichwohl der IR-Anteil des Therapielasers und dessen Darstellung über die Beobachtungseinheit vollständig genutzt werden kann. Eine schematisierte Darstellung eines Teils des diesbezüglichen Strahlenganges ist der Fig. 11a zu entnehmen. Ein bevorzugtes Transmissionsdiagramm des im Beleuchtungsstrahlengang eingesetzten Filters 13 ist der Fig. 11b zu entnehmen.

### Bezugszeichenliste

- 1: Punktlichtquelle als Halogen oder Xenonlampe
- 2: Kondensor 2-linsiges optisches System
- 3: Beleuchtungsblende
- 3': Beobachtungsblende
- 4: Beleuchtungsoptik
- 4': Beobachtungsoptik
- 5: Filteranordnung
- 6: Vorsatzlinse
- 7: Ophthalmoskopierlinse
- 8: CCD-Sensor verschiebbar (Farb-s/w-Kamera)
- 9: Auge
- 10: Austauschbaugruppe
- 11: Umlenkspiegel, Teilerspiegel
- 12: Therapielaseradapter
- 13: Schutzfilter
- 14: Blendenanordnung
- 15: Blendenspalt-Paar mit geringer Blendenspaltbreite
- 16: Blendenspalt-Paar mit großer Blendenspaltbreite
- 17: Federsystem
- 18: Induktionsmechanismus
- 19: lichtempfindliche Fläche der Beobachtungseinrichtung
- 20: Blendenspalt
- 21,22: Umlenkelemente, Umlenkspiegel, Strahlteiler
- 23: Shutter-Blende
- 24: Markierungseinrichtung
- 25: Irisblende, Display, Maske
- 26: Abbildungseinheit
- A: Beleuchtungs-Abbildungsebene
- A': Beobachtungs-Abbildungsebene
- AM: Mittenachse
- B: Zwischenbildebene Ophthalmoskopierlinse
- F: Fokuslinse
- L1: Therapielaser
- L2: Therapielaser

## Patentansprüche

1. Ophthalmoskop zur Untersuchung des Augenhintergrundes (9') eines Patienten (9) mit
- wenigstens einer, wenigstens einen Beleuchtungsstrahl erzeugenden Beleuchtungseinrichtung (1) sowie einer der Beleuchtungseinrichtung (1) zuordenbaren Abbildungsoptik (A_{Bl}), die den Beleuchtungsstrahl über eine Zwischenbildebene (B) auf den Augenhintergrund (9') des Patienten (9) abbildet,
- wenigstens einer Beobachtungseinrichtung (8) sowie einer der Beobachtungseinrichtung (8) zuordenbaren Abbildungsoptik (A_{Bo}), die einen durch Reflexion des Beleuchtungsstrahls am Augenhintergrund (9') erzeugten Beobachtungsstrahl über die Zwischenbildebene (B) in die Beobachtungseinrichtung (8) abbildet,
sowie
- einer im Beleuchtungs- und Beobachtungsstrahl eingebrachten Blendenspaltanordnung (3, 3') mit je wenigstens einem im Beleuchtungs- und Beobachtungsstrahl einbringbaren Blendenspalt, die relativ zu dem Beleuchtungs- und Beobachtungsstrahl synchron schwingend gelagert sind,
**dadurch gekennzeichnet, dass** über wenigstens eine optische Einheit (6) die Zwischenbildebene (B) ins Unendliche abbildbar ist,
dass die ins unendliche abgebildete Zwischenbildebene (B) in eine Abbildungsebene (A') des Beobachtungsstrahls abbildbar ist, in der die Beobachtungseinrichtung (8) vorgesehen ist, und
dass die optische Einheit (6) im Strahlengang des Beobachtungsstrahls der Zwischenbildebene (B) nachgeordnet ist und vom Beleuchtungs- als auch vom Beobachtungsstrahl durchsetzt wird.

2. Ophthalmoskop nach Anspruch 1,
**dadurch gekennzeichnet, dass** die Blendenspaltanordnung (3, 3') in Strahlrichtung des Beobachtungsstrahls unmittelbar vor der Abbildungsebene (A, A') vorgesehen ist.

3. Ophthalmoskop nach Anspruch 1 oder 2,
**dadurch gekennzeichnet, dass** die Beobachtungseinrichtung (8) ein CCD-Sensor ist.

4. Ophthalmoskop nach einem der Ansprüche 1 bis 3,
**dadurch gekennzeichnet, dass** der Beleuchtungsstrahl die Blendenspaltanordnung (3, 3') parallel zum Beobachtungsstrahl durchsetzt.

5. Ophthalmoskop nach einem der Ansprüche 1 bis 4,
**dadurch gekennzeichnet, dass** im Strahlengang längs des Beleuchtungs- und Beobachtungsstrahls zwischen dem Patienten (9) und der Zwischenbildebene (B) eine optische Abbildungseinheit (7) vorgesehen ist, die den Augenhintergrund in die Zwischenbildebene (B) sowie den Beleuchtungsstrahl auf den Augenhintergrund abbildet.

6. Ophthalmoskop nach Anspruch 5,
**dadurch gekennzeichnet, dass** die optische Abbildungseinheit (7) eine Ophthalmoskopierlinse ist.

7. Ophthalmoskop nach einem der Ansprüche 1 bis 6,
**dadurch gekennzeichnet, dass** die optische Einheit (6) wenigstens ein Achromat ist.

8. Ophthalmoskop nach einem der Ansprüche 1 bis 7,
**dadurch gekennzeichnet, dass** die optische Einheit (6) axial zum ins Unendliche gerichteten Beobachtungsstrahl skalierbar verschiebbar ist.

9. Ophthalmoskop nach einem der Ansprüche 1 bis 8,
**dadurch gekennzeichnet, dass** längs des Beobachtungsstrahls eine Optikeinheit (4') vorgesehen ist, die die ins Unendliche abgebildete Zwischenbildebene (B) in die Abbildungsebene (A') auf die Beobachtungseinrichtung (8) abbildet.

10. Ophthalmoskop nach einem der Ansprüche 1 bis 9,
**dadurch gekennzeichnet, dass** längs des Beleuchtungsstrahls eine Optikeinheit (4) vorgesehen ist, die die ins Unendliche abgebildete Zwischenbildebene (B) auf eine homogen ausgeleuchtete Fläche innerhalb der Abbildungsebene (A) abbildet.

11. Opthalmoskop nach Anspruch 9 und 10,
**dadurch gekennzeichnet, dass** die Optikeinheit (4) innerhalb des Beleuchtungsstrahls und die Optikeinheit (4') innerhalb des Beobachtungsstrahls in der optischen Wirkung identisch und/oder einstückig ausgebildet sind.

12. Ophthalmoskop nach einem der Ansprüche 1 bis 11,
**dadurch gekennzeichnet, dass** der Beleuchtungseinrichtung (1) eine Beleuchtungsoptik (2) zugeordnet ist, die den Beleuchtungsstrahl am Ort der Abbildungsebene (A) als homogen ausgeleuchtete Fläche abbildet.

13. Ophtalmoskop nach Anspruch 12,
**dadurch gekennzeichnet, dass** die Beleuchtungsoptik (2) ein Kondensorsystem ist.

14. Ophthalmoskop nach einem der Ansprüche 1 bis 13,
**dadurch gekennzeichnet, dass** der Beleuchtungs- und Beobachtungsstrahl im Bereich der ins Unendlich abgebildeten Zwischenbildebene (B) parallel verlaufen.

15. Ophthalmoskop nach einem der Ansprüche 1 bis 14,
**dadurch gekennzeichnet, dass** die in die Zwischenbildebene (B) abgebildeten Beobachtungs- und Beleuchtungsstrahlen einen Winkel 2α einschließen, und
dass die Winkelhalbierende des Winkels 2α parallel zum Strahlengang des Beleuchtungs- und Beobachtungsstrahls im Bereich der ins Unendliche abgebildeten Zwischenbildebene (B) verläuft.

16. Ophthalmoskop nach einem der Ansprüche 10 bis 15,
**dadurch gekennzeichnet, dass** die Optikeinheiten (4, 4') längs der Beobachtungs- und Beleuchtungsstrahlen mit einer Filtereinheit kombinierbar sind.

17. Ophthalmoskop nach einem der Ansprüche 1 bis 16,
**dadurch gekennzeichnet, dass** innerhalb der Zwischenbildebene (B) wenigstens eine längs zum Beobachtungs- und/oder Beleuchtungsstrahlengang verschiebbare, austauschbare optische Baugruppe eingebracht ist.

18. Ophthalmoskop nach Anspruch 17,
**dadurch gekennzeichnet, dass** die optische Baugruppe eine Glasplatte mit Markierungen, eine Blendenanordnung, eine Filteranordnung und/oder eine Durchlichtprojektionseinrichtung ist.

19. Ophthalmoskop nach einem der Ansprüche 1 bis 18,
**dadurch gekennzeichnet, dass** die Blendenspaltanordnung (3, 3') wenigstens zwei Blendenspalt-Paare (15, 16) vorsieht, von denen die Blendenspalte jedes Blendenspalt-Paars über identische Spaltbreiten verfügen und einzeln jeweils dem Beleuchtungs- (Bl) und dem Beobachtungsstrahl (Bo) zuordenbar sind,
dass die Blendenspalte der wenigstens zwei Blendenspalt-Paare über unterschiedlich große Spaltbreiten verfügen, und
dass die Blendenspalte der einzelnen Blendenspalt-Paare durch Verschieben der Blendenanordnung jeweils in den Beleuchtungs- und Beobachtungsstrahl bringbar sind.

20. Ophthalmoskop nach einem der Ansprüche 1 bis 19,
**dadurch gekennzeichnet, dass** im Bereich der ins Unendliche abgebildeten Zwischenbildebene (B) ein Spiegel (11) vorgesehen ist, über den ein weiterer Strahlengang z.B. ein Therapielaser längs zu dem Beleuchtungs- und/oder Beobachtungsstrahl einkoppelbar ist.

21. Ophthalmoskop nach einem der Ansprüche 1 bis 19,
**dadurch gekennzeichnet, dass** im Bereich zwischen der Zwischenbildebene (B) und der ins Unendliche abgebildeten Zwischenbildebene (B) ein Spiegel (11) vorgesehen ist, über den ein weiterer Strahlengang z.B. ein Therapielaser längs zu dem Beleuchtungs- und/oder Beobachtungsstrahl einkoppelbar ist.

22. Ophthalmoskop nach einem der Ansprüche 1 bis 19,
**dadurch gekennzeichnet, dass** im Bereich zwischen der Zwischenbildebene (B) und der ins Unendliche abgebildeten Zwischenbildebene (B) oder im Bereich der ins Unendliche abgebildeten Zwischenbildebene (B) ein Teilerspiegel (11) vorgesehen ist, über den ein weiterer Strahlengang, z.B. ein Therapielaser in den Beleuchtungs- und/oder Beobachtungsstrahl einkoppelbar ist.

23. Ophthalmoskop nach einem der Ansprüche 20 bis 22,
**dadurch gekennzeichnet, dass** der Spiegel bzw. der Teilerspiegel als Scanning Spiegel ausgebildet ist, der um wenigstens zwei orthogonale Raumachsen schwenkbar ist.

24. Ophthalmoskop einem der Ansprüche 20 bis 22,
**dadurch gekennzeichnet, dass** der weitere Strahlengang über die optische Einheit (6) in die Zwischenbildebene (B) fokussierbar ist.

25. Ophthalmoskop nach einem der Ansprüche 1 bis 23,
**dadurch gekennzeichnet, dass** sich im Beobachtungsstrahl ein fest eingebauter oder einschwenkbarer Schutzfilter (5) zur Sicherheit für die Beobachtungseinrichtung vorgesehen ist.

26. Ophthalmoskop nach Anspruch 1,
**dadurch gekennzeichnet, dass** die der Beleuchtungseinrichtung (1) zuordenbare Abbildungsoptik (A_{Bl}) folgende optische Komponenten aufweist:
eine Beleuchtungsoptikeinheit (2) zur Abbildung der Beleuchtungseinrichtung (1) in die Abbildungsebene (A),
eine Optikeinheit (4), die den aus der Abbildungsebene (A) austretenden Beleuchtungsstrahl ins Unendliche abbildet,
eine optische Einheit (6), die den ins Unendliche abgebildeten Beleuchtungsstrahl (Bl) auf die Zwischenbildebene (B) abbildet sowie eine optische Abbildungseinheit (7), die die Zwischenbildebene (B) auf den Augenhintergrund (9') des Patienten (9) abbildet, und
dass die der Beobachtungseinrichtung (8) zuordenbare Abbildungsoptik (A_{Bo}) in Strahlrichtung des Beobachtungstrahls (Bo) ausgehend vom Augenhintergrund (9') des Patienten (9) folgende optische Komponenten aufweist:
die Abbildungseinheit (7), die den Augenhintergrund auf die Zwischenbildebene (B) abbildet,
die optische Einheit (6), die die Zwischenbildebene (B) ins Unendliche abbildet sowie eine Optikeinheit (4'), die die ins Unendliche abgebildete Zwischenbildebene (B) in die Abbildungsebene (A') abbildet.

27. Ophthalmoskop nach einem der Ansprüche 19 bis 26,
**dadurch gekennzeichnet, dass** die in der Abbildungsebene (A') befindliche Beobachtungseinrichtung eine Detektorfläche mit einer Flächenerstreckung vorsieht, längs der der dem Beobachtungsstrahl zugeordnete Blendenspalt derart schwingt, dass der Blendenspalt eines ersten Blendenspalt-Paares einen Schwingungsumkehrpunkt aufweist, bei dem der Blendenspalt vollständig ausserhalb der Detektorfläche liegt, und
dass der Blendenspalt eines zweiten Blendenspalt-Paares einen Schwingungsumkehrpunkt aufweist, bei dem der Blendenspalt vollständig innerhalb der Detektorfläche liegt.

28. Ophthalmoskop nach Anspruch 27,
**dadurch gekennzeichnet, dass** die Spaltbreite des Blendenspalts des ersten Blendenspalt-Paares kleiner als die des Blendenspalts des zweiten Blendenspalt-Paares ist.

29. Ophthalmoskop nach Anspruch 27 oder 28,
**dadurch gekennzeichnet, dass** die Spaltbreite des Blendenspalts des ersten Blendenspalt-Paares kleiner ist als Hälfte der Flächenerstreckung der Detektorfläche, längs der der Blendenspalt schwingt, und
dass die Spaltbreite des Blendenspalts des zweiten Blendenspalt-Paares gleich oder gößer der Hälfte der Flächenerstreckung der Detektorfläche ist.

30. Ophthalmoskop nach einem der Ansprüche 1 bis 29,
**dadurch gekennzeichnet, dass** zwei räumlich getrennte Beobachtungseinrichtungen mit zugehörigen Beobachtungstrahlengängen vorgesehen sind, die räumlich voneinander getrennt über eine erste und zweite Abbildungsoptik (A'_{Bo}) bzw. (A"_{Bo}) den Augenhintergrund (9') über die Zwischenbildebene (B) auf die jeweilige Beobachtungseinrichtung abbilden.

31. Ophthalmoskop nach einem der Ansprüche 1 bis 29,
**dadurch gekennzeichnet, dass** zwei räumlich getrennte Beobachtungstrahlengänge vorgesehen sind, die räumlich voneinander getrennt über eine erste und zweite Abbildungsoptik (A'_{Bo}) bzw. (A"_{Bo}) den Augenhintergrund (9') über die Zwischenbildebene (B) auf eine einzige Beobachtungseinrichtung abbilden und
dass eine Teileroptik vorgesehen ist, durch die beide Beobachtungsstrahlengänge getrennt voneinander, abwechselnd zeitlich aufeinanderfolgend auf die Beobachtungseinheit auftreffen.

32. Ophthalmoskop nach Anspruch 30 oder 31,
**dadurch gekennzeichnet, dass** das Opthalmoskop zur stereoskopischen Beobachtung des Augenhintergrundes geeignet ist.

33. Ophthalmoskop nach einem der Ansprüche 1 bis 32,
**dadurch gekennzeichnet, dass** eine Verschwenkvorrichtung vorgesehen ist, die das Opthlamoskop in einer den Augenhintergrund schneidenden Ebene um einen vorgebbaren Winkel aus einer ersten in eine zweite Position verschwenkt, und dass eine optische Markiereinheit vorgesehen ist, die unabhängig von der Position eine feste, ortsunveränderliche Blickrichtung für den Patienten definiert.

34. Ophthalmoskop nach einem der Ansprüche 1 bis 33,
**dadurch gekennzeichnet, dass** die optische Einheit (6) längs zum Beobachtungsstrahl derart verlagerbar ist, dass eine Verlagerung der optischen Einheit (6) räumlich auflösbar ist.

35. Ophthalmoskop nach Anspruch 34,
**dadurch gekennzeichnet, dass** die optische Einheit (6) mittels eines Schrittmotors verlagerbar ist.

36. Ophthalmoskop nach einem der Ansprüche 1 bis 35,
**dadurch gekennzeichnet, dass** im Beleuchtungsstrahl ein einschwenkbares, wellenlängenselektives Schutzfilter vorgesehen ist.

37. Ophthalmoskop nach Anspruch 36,
**dadurch gekennzeichnet, dass** der Schutzfilter sichtbares Licht weitgehend absobiert und IR-Licht transmittiert.

38. Ophthalmoskop nach Anspruch 36 oder 37,
**dadurch gekennzeichnet, dass** der Schutzfilter ein Langpassinterferenzfilter ist.

## Claims

1. An ophthalmoscope for examining the fundus (9') of a patient's eye, having
- at least one illumination means (1), which generates at least one illumination beam, and one imaging optic (A_{Bl}) assignable to said illumination means (1), said imaging optic (A_{Bl}) imaging said illumination beam onto said fundus (9') of a patient's eye (9) via an intermediate focal plane (B),
- at least one observation means (8) and an imaging optic (A_{Bo}) assignable to said observation means (8), said imaging optic (A_{Bo}) imaging an observation beam generated by reflection of said illumination beam at said fundus (9') via said intermediate plane (B) in said observation means (8),
and
- a diaphragm slit arrangement (3,3') placed in said illumination beam and said observation beam, said diaphragm slit arrangement (3,3') having at least one diaphragm slit, respectively, placeable in said illumination beam path and said observation beam path, said diaphragm slits being mounted in a synchronously oscillating manner relative to said illumination beam and said observation beam,
**wherein** said intermediate focal plane (B) is imageable to infinity via at least one optical unit (6) and said intermediate focal plane imaged to infinity is imageable in an imaging plane (A') of said observation beam in which said observation means (8) is provided and
said optical unit (6) is provided in the beam path of said observation beam downstream of said intermediate focal plane (B) and is passed through by said illumination beam and said observation beam.

2. The ophthalmoscope according to claim 1,
wherein said diaphragm slit arrangement (3,3') is provided immediately before said imaging plane (A, A') in the beam direction of said observation beam.

3. The ophthalmoscope according to claim 1 or 2,
wherein said observation means (8) is a CCD sensor.

4. The ophthalmoscope according to one of the claims 1 to 3,
wherein said illumination beam passes through said diaphragm arrangement (3,3') parallel to said observation beam.

5. The ophthalmoscope according to one of the claims 1 to 4,
**wherein** an optical imaging unit (7), which images said fundus in said intermediate plane (B) as well as said illumination beam on said fundus, is provided in the beam path in longitudinal direction of said illumination beam and said observation beam between said patient (9) and said intermediate focal plane (B).

6. The ophthalmoscope according to claim 5,
wherein said optical imaging unit (7) is an ophthalmoscope lens.

7. The ophthalmoscope according to one of the claims 1 to 6,
**wherein** said optical unit (6) is at least one achromat.

8. The ophthalmoscope according to one of the claims 1 or 7,
**wherein** said optical unit (6) is movable in scaleable steps axially to the observation beam which is directed to infinity.

9. The ophthalmoscope according to one of the claims 1 to 8,
wherein in longitudinal direction of said observation beam, an optic unit (4') is provided which images said intermediate focal plane (B) imaged to infinity in said imaging plane (A') on said observation means (8).

10. The ophthalmoscope according to one of the claims 1 to 9,
wherein in longitudinal direction of said illumination beam, an optic unit (4) is provided, which images said intermediate focal plane (B) imaged to infinity on a uniformly lit area within said imagining plane (A).

11. The ophthalmoscope according to claim 9 and 10,
**wherein** said optic unit (4) in said illumination beam and said optic unit (4') in said observation beam are designed identical in optical effect and/or one-piece.

12. The ophthalmoscope according to one of the claims 1 to 11,
wherein to said illumination means (1) is assigned an illumination optic (2), which images said illumination beam at the site of said imaging plane (A) as a uniformly lit area.

13. The ophthalmoscope according to claim 12,
**wherein** said illumination optic (2) is a condenser system.

14. The ophthalmoscope according to one of the claims 1 to 13,
wherein said illumination beam and said observation beam run parallel in the region of said intermediate focal plane (B) imaged to infinity.

15. The ophthalmoscope according to one of the claims 1 to 14,
**wherein** said observation beam and said illumination beam imaged in said intermediate focal plane (B) form an angle 2α and the bisecting line of said angle 2α runs parallel to the beam path of said illumination beam and said observation beam in the region of said intermediate focal plane (B) imaged to infinity.

16. The ophthalmoscope according to one of the claims 10 to 15,
**wherein** said optic units (4,4') are combinable with a filter unit in longitudinal direction of said observation beams and illumination beams.

17. The ophthalmoscope according to one of the claims 1 to 16,
**wherein** at least one group of replaceable optical components moveable longitudinally to said observation beam path and/or said intermediate beam path is placed in said intermediate focal plane (B).

18. The ophthalmoscope according to claim 17,
**wherein** said group of optical components is a glass plate with markings, a diaphragm arrangement, a filter arrangement and/or a rear projection means.

19. The ophthalmoscope according to one of the claims 1 to 18,
**wherein** said diaphragm slit arrangement (3,3') is provided with at least two pairs of diaphragm slits (15,16), of which the diaphragm slits of each said pair of diaphragm slits have identical slit widths and are singly assignable to said illumination beam (Bl) and said observation beam (Bo) respectively, the diaphragm slits of at least two pairs of said diaphragm slits have slit widths of different sizes, and the diaphragm slits of said single pairs of diaphragm slits are placeable in said illumination beam and said observation beam respectively by moving said diaphragm arrangement.

20. The ophthalmoscope according to one of the claims 1 to 19,
wherein in the region of said intermediate focal plane (B) imaged to infinity, a mirror (11) is provided via which an additional beam path, for example a therapeutic laser, can be coupled in longitudinally to said illumination beam and said observation beam.

21. The ophthalmoscope according to one of the claims 1 to 19,
**wherein** in the region between said intermediate focal plane (B) and said intermediate focal plane (B) imaged to infinity a mirror (11) is provided via which an additional beam path, for example a therapeutic laser, can be coupled in longitudinally to said illumination beam and/or said observation beam.

22. The ophthalmoscope according to one of the claims 1 to 19,
**wherein** a splitter mirror (11), via which an additional beam path, for example a therapeutic laser, can be coupled into said illumination beam and/or said observation beam, is provided in said region between said intermediate focal plane (B) and said intermediate focal plane (B) imaged to infinity or in said region of said intermediate focal plane imaged to infinity.

23. The ophtalmoscope according to one of the claims 20 to 22,
**wherein** said mirror respectively said splitter mirror is designed as a scanning mirror which is swingable about at least two orthogonal spatial axes.

24. The ophthalmoscope according to one of the claims 20 to 22,
**wherein** said additional beam path is focusable via said optical unit (6) into said intermediate focal plane (B).

25. The ophthalmoscope according to one of the claims 1 to 23,
**wherein** a fixed, built-in protective filter (5) or a protective filter (5) which can be swing into said observation beam is provided in said observation beam for safeguarding said observation means.

26. The ophthalmoscope according to claim 1,
**wherein** said imaging optic (A_{Bl}) assignable to said illumination means (1) is provided with the following optical components:
an illumination optic unit (2) for imaging said illumination means (1) in said imaging plane (A),
an optic unit (4), which images the illumination beam emerging from said imaging plane (A) to infinity,
an optical unit (6), which images said illumination beam (BI) imaged to infinity on said intermediate focal plane (B) including
an optical imaging unit (7), which images said intermediate focal plane (B) on said fundus (9') of said patient's eye (9), and
said imaging optic (A_{Bo}) assignable to said observation means (8) is provided with the following optical components in the beam direction of said observation beam (Bo) starting from said fundus (9') of said patient's eye (9): said imaging unit (7), which images said fundus (9') onto said intermediate focal plane (B),
said optical unit (6), which images said intermediate focal plane (B) to infinity, and an optic unit (4'), which images said intermediate plane (B) imaged to infinity in said imaging plane (A').

27. The ophthalmoscope according to one of the claims 19 to 26,
**wherein** said observation means located in said imaging plane (A') is provided with a detector area having a span along which the diaphragm slit assigned to said observation beam oscillates in such a manner that the diaphragm slit of a first pair of diaphragm slits has an oscillation turning point in which said diaphragm slit lies completely
outside said detector area,
the diaphragm slit of a second pairs of diaphragm slits has an oscillation turning point in which said diaphragm slit lies completely within said detector area.

28. The ophthalmoscope according to claim 27,
wherein the slit width of said diaphragm slit of said first pair of diaphragm slits is smaller than the width of the slits of said second pair of diaphragm slits.

29. The ophthalmoscope according to claim 27 to 28,
**wherein** the slit width of the diaphragm slits of said first pair of diaphragm slits is smaller than half of the span of said detector area, along which said diaphragm slit oscillates, and
the slit width of said diaphragm slits of said second pairs of diaphragm slits is the same size as or larger than half of the span of said detector area.

30. The ophthamoscope according to one of the claims 1 to 29,
wherein two spatially separate observation means are provided with corresponding observation beam paths, which spatially separated from each other image said fundus (9') via said intermediate focal plane (B) on the respective observation means via a first and a second imaging optic (A'_{Bo}) respectively (A"_{Bo}).

31. The ophtalmoscope according to one of the claims 1 to 29,
wherein two spatially separate observation beam paths are provided, which spatially separated from each image said fundus (9') via said intermediate focal plane (B) on a single observation means via a first and a second imaging optic (A'_{Bo}) respectively (A"_{Bo}) and
a splitter optic is provided by means of which said two observation beam paths impinge on said observation means separated from each other, altemately in succession.

32. The ophthalmoscope according to claim 30 or 31,
**wherein** said ophthalmoscope is suited for stereoscopic observation of the fundus.

33. The ophthalmoscope according to one of the claims 1 to 32,
**wherein** a swing mechanism is provided which swings said ophthalmoscope a given angle from a first position into a second position into a plane intersecting the fundus, and an optical marking unit is provided which defines, independent of the position, an unchanging, site-fixed viewing direction for the patient.

34. The ophthalmoscope according to one of the claims 1 to 33,
**wherein** the position of said optical unit (6) can be moved in the longitudinal direction of the observation beam in such a manner that moving said optical unit (6) is measurable.

35. The ophthalmoscope according to claim 34,
**wherein** the position of said optical unit (6) can be movable by means of a step-by-step motor.

36. The opthalmoscope according to one of the claims 1 to 35,
**wherein** a wave-length selective protective filter which can be swung into the illumination beam is provided.

37. The ophthalmoscope according to claim 36,
**wherein** said protective filter largely absorbs visible light and transmits infrared light.

38. The ophthalmoscope according to claim 36 or 37,
**wherein** said protective filter is a long pass interference filter.

## Revendications

1. Ophtalmoscope pour l'examen du fond d'oeil (9') d'un patient comportant :
- au moins un dispositif d'éclairage (1) produisant au moins un rayon d'éclairage ainsi qu'une optique de représentation (A_{Bl}) pouvant être associée au dispositif d'éclairage (1), laquelle reproduit le rayon d'éclairage par le biais d'un plan d'image intermédiaire (B) sur le fond d'oeil (9') du patient (9),
- au moins un dispositif d'observation (8) ainsi qu'une optique de représentation (A_{Bo}) pouvant être associée au dispositif d'observation (8), laquelle reproduit un rayon produit par réflexion du rayon d'éclairage sur le fond d'oeil (9') par le biais du plan d'image intermédiaire (B) dans le dispositif d'observation (8),
ainsi
- qu'un dispositif de fentes à diaphragmes (3, 3') installé dans le rayon d'éclairage et d'observation, comprenant un moins une fente à diaphragme et pouvant être placé dans un rayon d'éclairage et d'observation, ces fentes étant posées en oscillation synchrone par rapport au rayon d'éclairage et d'observation,
**caractérisé en ce que** le plan d'image intermédiaire (B) peut être représenté dans l'infini par au moins une unité optique (6),
que le plan d'image intermédiaire (B) représenté dans l'infini peut être représenté dans un plan de représentation (A') du rayon d'observation où est prévu le dispositif d'observation (8) et
que l'unité optique (6) est disposée en aval du plan d'image intermédiaire (B) dans le parcours optique du rayon d'observation et est traversée à la fois par le rayon d'éclairage et le rayon d'observation.

2. Ophtalmoscope selon la revendication 1,
**caractérisé en ce que** le dispositif de fentes à diaphragme (3, 3') est prévu dans la direction de rayonnement du rayon d'observation juste avant le plan de représentation (A, A').

3. Ophtalmoscope selon la revendication 1 ou 2,
**caractérisé en ce que** le dispositif d'observation (8) est un capteur CCD (à couplage de charge).

4. Ophtalmoscope selon l'une quelconque des revendications 1 à 3,
**caractérisé en ce que** le rayon d'éclairage traverse le dispositif de fentes à diaphragmes (3, 3') parallèlement au rayon d'observation.

5. Ophtalmoscope selon l'une quelconque des revendications 1 à 4,
**caractérisé en ce que**, dans le parcours optique, le long du rayon d'éclairage et d'observation, il est prévu, entre le patient (9) et le plan d'image intermédiaire (B), une unité de représentation optique (7) qui représente le fond d'oeil dans le plan d'image intermédiaire (B) ainsi que le rayon d'éclairage sur le fond d'oeil.

6. Ophtalmoscope selon la revendication 5,
**caractérisé en ce que** l'unité de représentation optique (7) est une lentille d'ophtalmoscopie.

7. Ophtalmoscope selon l'une quelconque des revendications 1 à 6,
**caractérisé en ce que** l'unité optique (6) est au moins un achromate.

8. Ophtalmoscope selon l'une quelconque des revendications 1 à 7,
**caractérisé en ce que** l'unité optique (6) est déplaçable de manière graduable axialement par rapport au rayon d'observation orienté vers l'infini.

9. Ophtalmoscope selon l'une quelconque des revendications 1 à 8,
**caractérisé en ce que**, le long du rayon d'observation, il est prévu une unité optique (4') qui reproduit le plan intermédiaire (B) reproduit dans l'infini dans le plan d'observation (A') du dispositif d'observation (8).

10. Ophtalmoscope selon l'une quelconque des revendications 1 à 9,
**caractérisé en ce que**, le long du rayon d'éclairage, il est prévu une unité optique (4) qui reproduit le plan intermédiaire (B) reproduit dans l'infini sur une surface éclairée de manière homogène dans le plan de représentation (A).

11. Ophtalmoscope selon les revendications 9 et 10,
**caractérisé en ce que** l'unité optique est réalisée à l'intérieur du rayon d'éclairage et que l'unité optique (4') est réalisée à l'intérieur du rayon d'observation de manière à avoir un effet optique identique et/ou en une pièce.

12. Ophtalmoscope selon l'une quelconque des revendications 1 à 11,
**caractérisé en ce qu'**au dispositif d'éclairage (1) est associée une optique d'éclairage (2) qui reproduit le rayon d'éclairage à l'emplacement du plan de représentation (A) se présentant comme une surface éclairée de manière homogène.

13. Ophtalmoscope selon la revendication 12,
**caractérisé en ce que** l'optique d'éclairage (2) est un système à condensateur.

14. Ophtalmoscope selon l'une quelconque des revendications 1 à 13,
**caractérisé en ce que** le rayon d'éclairage et le rayon d'observation s'étendent parallèlement et au niveau du plan d'image intermédiaire représenté dans l'infini (B).

15. Ophtalmoscope selon l'une quelconque des revendications 1 à 14,
**caractérisé en ce que** les rayons d'éclairage et d'observation représentés dans le plan d'image intermédiaire (B) décrivent un angle 2α et que la bissectrice de l'angle 2α s'étend parallèlement au parcours optique du rayon d'éclairage et d'observation au niveau du plan d'image intermédiaire représenté dans l'infini (B).

16. Ophtalmoscope selon l'une quelconque des revendications 10 à 15,
**caractérisé en ce que** les unités optiques (4, 4'), le long des rayons d'observation et d'éclairage, sont combinables à une unité de filtrage.

17. Ophtalmoscope selon l'une quelconque des revendications 1 à 16,
**caractérisé en ce que**, dans le plan d'image intermédiaire (B), est installé au moins un agrégat optique échangeable et déplaçable longitudinalement par rapport au parcours optique d'observation et d'éclairage.

18. Ophtalmoscope selon la revendication 17,
**caractérisé en ce que** l'agrégat optique est une plaque de verre comportant des marquages, un dispositif de diaphragmes, un dispositif de filtrage et/ou un dispositif de projection de lumière passant à travers.

19. Ophtalmoscope selon l'une quelconque des revendications 1 à 18,
**caractérisé en ce que** le dispositif de fentes à diaphragmes (3, 3') comprend au moins deux paires de fentes à diaphragmes (15, 16) dont les fentes de diaphragme de chaque paire de fentes à diaphragmes présentent des largeurs de fentes identiques et peuvent être associées individuellement respectivement au rayon d'éclairage (Bl) et au rayon d'observation (Bo), que les fentes de diaphragmes des au moins deux paires de fentes à diaphragmes présentent des largeurs de fente de dimensions différentes et
que les fentes de diaphragme des différentes paires de fentes à diaphragmes peuvent être amenées, en déplaçant le dispositif à diaphragmes, respectivement dans le rayon d'éclairage et d'observation.

20. Ophtalmoscope selon l'une quelconque des revendications 1 à 19,
**caractérisé en ce qu'**au niveau du plan d'image intermédiaire (B) reproduit dans l'infini, il est prévu un miroir (11) qui permet d'engager un autre parcours optique, par exemple un laser thérapeutique, longitudinalement par rapport au rayon d'éclairage et/ou d'observation.

21. Ophtalmoscope selon l'une quelconque des revendications 1 à 19,
**caractérisé en ce que**, dans la zone située entre le plan d'image intermédiaire (B) et le plan d'image intermédiaire (B) reproduit dans l'infini, il est prévu un miroir (11) qui permet d'engager un autre parcours optique, par exemple un laser thérapeutique, longitudinalement par rapport au rayon d'éclairage et/ou d'observation.

22. Ophtalmoscope selon l'une quelconque des revendications 1 à 19,
**caractérisé en ce que**, dans la zone située entre le plan d'image intermédiaire (B) et le plan d'image intermédiaire (B) reproduit dans l'infini ou dans la zone du plan d'image intermédiaire (B) reproduit dans l'infini, il est prévu un miroir diviseur (11) qui permet d'engager un autre parcours optique, par exemple un laser thérapeutique, dans le rayon d'éclairage et/ou d'observation.

23. Ophtalmoscope selon l'une quelconque des revendications 20 à 22,
**caractérisé en ce que** le miroir ou le miroir diviseur est réalisé sous forme d'un miroir de balayage qui peut pivoter autour d'au moins deux axes spatiaux orthogonaux.

24. Ophtalmoscope selon l'une quelconque des revendications 20 à 22,
**caractérisé en ce que** l'autre parcours optique peut être focalisé par l'unité optique (6) dans le plan d'image intermédiaire (B).

25. Ophtalmoscope selon l'une quelconque des revendications 1 à 23,
**caractérisé en ce qu'**il est prévu dans le rayon d'observation un filtre protecteur monté fixement ou pivotable à l'intérieur (5) et servant de sécurité pour le dispositif d'observation.

26. Ophtalmoscope selon la revendication 1,
**caractérisé en ce que** l'optique de reproduction (A_{Bl}) pouvant être associée au dispositif d'éclairage (1) comprend les composants optiques suivants :
une unité optique d'éclairage (2) pour la représentation du dispositif d'éclairage (1) dans le plan de représentation (A),
une unité optique (4) qui reproduit dans l'infini le rayon d'éclairage sortant du plan de représentation (A) ,
une unité optique qui reproduit le rayon d'éclairage (Bl) reproduit dans l'infini sur le plan d'image intermédiaire (B)
ainsi qu'une unité de représentation optique (7) qui reproduit le plan d'image intermédiaire (B) sur le fond d'oeil (9') du patient (9), et
que l'optique de représentation (A_{Bo}) pouvant être associée au dispositif d'observation (B), dans la direction de rayonnement du rayon d'observation (Bo), en partant du fond d'oeil (9') du patient (9), présente les composants optiques suivants :
l'unité de représentation (7) qui reproduit le fond d'oeil sur le plan d'image intermédiaire (B),
l'unité optique (6) qui reproduit le plan d'image intermédiaire (B) dans l'infini ainsi qu'une unité optique (4') qui reproduit le plan d'image intermédiaire (B) reproduit dans l'infini dans le plan de reproduction (A').

27. Ophtalmoscope selon l'une quelconque des revendications 19 à 26,
**caractérisé en ce que** le dispositif d'observation se trouvant dans le plan de reproduction (A') comprend une surface de détection avec une extension surfacique le long de laquelle la fente à diaphragme associée au rayon d'observation oscille de manière à ce que la fente à diaphragme d'une première paire de fentes à diaphragmes présente un point d'inversion d'oscillation auquel la fente à diaphragme se trouve entièrement à l'extérieur de la surface de détection, et
que la fente à diaphragme d'une deuxième paire de fentes à diaphragmes présente un point d'inversion d'oscillation auquel la fente à diaphragme se trouve entièrement à l'intérieur de la surface de détection.

28. Ophtalmoscope selon la revendication 27,
**caractérisé en ce que** la largeur de fente de la fente à diaphragme de la première paire de fentes à diaphragmes est plus petite que celle de la fente à diaphragme de la deuxième paire de fentes à diaphragmes.

29. Ophtalmoscope selon la revendication 27 ou 28,
**caractérisé en ce que** la largeur de fente de la fente à diaphragme de la première paire de fentes à diaphragmes est plus petite que la moitié de l'extension surfacique de la surface de détection le long de laquelle la fente à diaphragme oscille, et que la largeur de fente de la fente à diaphragme de la deuxième paire de fentes à diaphragmes est supérieure ou égale à la moitié de l'extension surfacique de la surface de détection.

30. Ophtalmoscope selon l'une quelconque des revendications 1 à 29,
**caractérisé en ce qu'**il est prévu deux dispositifs d'observation séparés dans l'espace avec des parcours optiques d'observation correspondants qui, étant séparés l'un de l'autre dans l'espace, grâce à une première et une deuxième optique de reproduction (A'Bo) ou (A'' Bo), reproduisent le fond d'oeil (9') par le biais du plan d'image intermédiaire (B) sur le dispositif d'observation respectif.

31. Ophtalmoscope selon l'une quelconque des revendications 1 à 29,
**caractérisé en ce qu'**il est prévu deux parcours optiques d'observation séparés l'un de l'autre dans l'espace qui, étant séparés l'un de l'autre dans l'espace, grâce à une première et une deuxième optique de reproduction (A'Bo) ou (A'' Bo), reproduisent le fond d'oeil (9') par le biais du plan d'image intermédiaire (B) sur un seul dispositif d'observation et qu'il est prévu une optique diviseuse grâce à laquelle les deux parcours optiques d'observation, séparément l'un de l'autre, arrivent alternativement et successivement dans le temps sur l'unité d'observation.

32. Ophtalmoscope selon la revendications 30 ou 31,
**caractérisé en ce que** l'ophtalmoscope est approprié pour une observation stéréoscopique du fond d'oeil.

33. Ophtalmoscope selon l'une quelconque des revendications 1 à 32,
**caractérisé en ce qu'**il est prévu un dispositif de pivotement qui fait pivoter l'ophtalmoscope dans un plan coupant le fond d'oeil, suivant un angle prescriptible, d'une première vers une deuxième position, et qu'il est prévu une unité de marquage optique qui, indépendamment de la position, définit pour le patient un sens de vision fixe dont l'emplacement ne change pas.

34. Ophtalmoscope selon l'une quelconque des revendications 1 à 33,
**caractérisé en ce que** l'unité optique (6) peut être décalée longitudinalement par rapport au rayon d'observation de manière à ce qu'un décalage de l'unité optique (6) dans l'espace soit définissable.

35. Ophtalmoscope selon la revendication 34,
**caractérisé en ce que** l'unité optique (6) peut être décalée au moyen d'un moteur pas à pas.

36. Ophtalmoscope selon l'une quelconque des revendications 1 à 35,
**caractérisé en ce qu'**il est prévu, dans le rayon d'éclairage, un filtre protecteur pivotable vers l'intérieur et sélectif de longueur d'ondes.

37. Ophtalmoscope selon la revendication 36,
**caractérisé en ce que** le filtre protecteur absorbe dans une large mesure de la lumière visible et transmet de la lumière IR.

38. Ophtalmoscope selon la revendication 36 ou 37,
**caractérisé en ce que** le filtre protecteur est un filtre à interférence passe-long.
